(19)

Europäisches
Patentamt
European
Patent Office
Office européen
des brevets

(11) **EP 3 604 525 B1**

(12) **EUROPEAN PATENT SPECIFICATION**

(45) Date of publication and mention
of the grant of the patent:
**10.03.2021  Bulletin 2021/10**

(51) Int Cl.:
***C12N 15/10*** *(2006.01)*     ***C40B 40/08*** *(2006.01)*

(21) Application number: **18186948.8**

(22) Date of filing: **02.08.2018**

(54) **METHOD FOR PROVIDING A DNA-ENCODED LIBRARY, DNA-ENCODED LIBRARY AND METHOD OF DECODING A DNA-ENCODED LIBRARY**

VERFAHREN ZUR BEREITSTELLUNG EINER DNA-CODIERTEN BIBLIOTHEK, DNA-CODIERTE BIBLIOTHEK UND VERFAHREN ZUR DECODIERUNG EINER DNA-CODIERTEN BIBLIOTHEK

PROCÉDÉ PERMETTANT DE FOURNIR UNE BIBLIOTHÈQUE ENCODÉE PAR ADN, BIBLIOTHÈQUE ENCODÉE PAR ADN ET PROCÉDÉ DE DÉCODAGE D'UNE BIBLIOTHÈQUE ENCODÉE PAR ADN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**05.02.2020   Bulletin 2020/06**

(73) Proprietor: **TU Dresden**
**01069 Dresden (DE)**

(72) Inventors:
 • **Zhang, Yixin**
  **01309 Dresden (DE)**
 • **Reddavide, Francesco**
  **01099 Dresden (DE)**
 • **Cui, Meiying**
  **01307 Dresden (DE)**
 • **Andrade, Helena**
  **01309 Dresden (DE)**
 • **Heiden, Stephan**
  **01187 Dresden (DE)**

(74) Representative: **Pfenning, Meinig & Partner mbB**
**Patent- und Rechtsanwälte**
**Theresienhöhe 11a**
**80339 München (DE)**

(56) References cited:
 **WO-A1-2014/065756     WO-A1-2017/075292**
 **WO-A1-2018/005720     WO-A2-2016/008822**
 **CN-A- 108 220 392**

 • **YIZHOU LI ET AL: "Quantitative PCR is a Valuable Tool to Monitor the Performance of DNA-Encoded Chemical Library Selections", CHEMBIOCHEM - A EUROPEAN JOURNAL OF CHEMICAL BIOLOGY., vol. 18, no. 9, 16 March 2017 (2017-03-16) , pages 848-852, XP055535160, DE ISSN: 1439-4227, DOI: 10.1002/cbic.201600626**
 • **RAPHAEL M. FRANZINI ET AL: "DNA-Encoded Chemical Libraries: Advancing beyond Conventional Small-Molecule Libraries", ACCOUNTS OF CHEMICAL RESEARCH, vol. 47, no. 4, 28 March 2014 (2014-03-28) , pages 1247-1255, XP055211954, ISSN: 0001-4842, DOI: 10.1021/ar400284t**
 • **J?RG SCHEUERMANN ET AL: "Dual-pharmacophore DNA-encoded chemical libraries", CURRENT OPINION IN CHEMICAL BIOLOGY, vol. 26, 1 June 2015 (2015-06-01), pages 99-103, XP055211951, ISSN: 1367-5931, DOI: 10.1016/j.cbpa.2015.02.021**
 • **MORENO WICHERT ET AL: "Dual-display of small molecules enables the discovery of ligand pairs and facilitates affinity maturation", NATURE CHEMISTRY, vol. 7, no. 3, 26 January 2015 (2015-01-26), pages 241-249, XP055184296, ISSN: 1755-4330, DOI: 10.1038/nchem.2158**
 • **FABIAN BULLER ET AL: "Drug discovery with DNA-encoded chemical libraries", BIOCONJUGATE CHEMISTRY, AMERICAN CHEMICAL SOCIETY, US, vol. 21, no. 9, 15 September 2010 (2010-09-15), pages 1571-1580, XP002695068, ISSN: 1043-1802, DOI: 10.1021/BC1001483 [retrieved on 2010-08-03]**

**(Cont. next page)**

**EP 3 604 525 B1**

- **ALIX I CHAN ET AL: "Novel selection methods for DNA-encoded chemical libraries", CURRENT OPINION IN CHEMICAL BIOLOGY, vol. 26, 1 June 2015 (2015-06-01), pages 55-61, XP055535155, GB ISSN: 1367-5931, DOI: 10.1016/j.cbpa.2015.02.010**
- Helena Andrade ET AL: "Modelling Large, Dynamic, and Heterogeneous Populations Using DNA Libraries", bioRxiv, 5 March 2018 (2018-03-05), XP055534080, DOI: 10.1101/276873 Retrieved from the Internet: URL:https://www.biorxiv.org/content/biorxiv/early/2018/03/05/276873.full.pdf
- **LUCA MANNOCCI ET AL: "20 years of DNA-encoded chemical libraries", CHEMICAL COMMUNICATIONS, ROYAL SOCIETY OF CHEMISTRY, vol. 47, no. 48, 28 December 2011 (2011-12-28), pages 12747-12753, XP002695069, ISSN: 1359-7345, DOI: 10.1039/C1CC15634A [retrieved on 2011-11-14]**
- **SCHEUERMANN J ET AL: "DNA-encoded chemical libraries for the discovery of MMP-3 inhibitors", BIOCONJUGATE CHEMISTRY, AMERICAN CHEMICAL SOCIETY, US, vol. 19, no. 3, 1 March 2008 (2008-03-01), pages 778-785, XP002737640, ISSN: 1043-1802, DOI: 10.1021/BC7004347 [retrieved on 2008-02-07]**
- **JEAN PIERRE DAGUER ET AL: "DNA-templated combinatorial assembly of small molecule fragments amenable to selection/amplification cycles", CHEMICAL SCIENCE, vol. 2, no. 4, 24 January 2011 (2011-01-24), page 625, XP055212512, ISSN: 2041-6520, DOI: 10.1039/c0sc00574f**
- **MIKE THOMPSON: "Adapting DNA-encoded library technology for fragment-based drug discovery", MEDICINAL CHEMISTRY, vol. 07, 1 June 2017 (2017-06-01), pages 83-83, XP055535198, DOI: 10.4172/2161-0444-C1-031**

SEQUENCE LISTING

<110> Technische Universitat Dresden

<120> Method for providing a DNA-encoded library, DNA-encoded library and method of decoding a DNA-encoded library

<130> 179P 3353

<160> 6

<170> BiSSAP 1.3.6

<210> 1
<211> 18
<212> DNA
<213> Artificial Sequence

<220>
<223> primer annealing to first part #1 of coding region I

<400> 1
gctgttccca cattgcgt          18

<210> 2
<211> 27
<212> DNA
<213> Artificial Sequence

<220>
<223> primer annealing to third part #3 of coding region I

<400> 2

ccttctggat tcggtcggag caccatc          27

<210> 3
<211> 27
<212> DNA
<213> Artificial Sequence

<220>
<223> primer annealing to first part #1 of coding region II

<400> 3
gatggtgctc cgaccgaatc cagaagg          27

<210> 4
<211> 34
<212> DNA
<213> Artificial Sequence

<220>
<223> primer annealing to third part #3 of coding region II

<400> 4
ggaggtgtag acgacagagt atttgactgt cagg          34

<210> 5
<211> 18
<212> DNA
<213> Artificial Sequence

<220>
<223> primer annealing to first part #1 of coding region III

<400> 5
cagatcgagc aactccac          18

<210> 6
<211> 18
<212> DNA
<213> Artificial Sequence

<220>
<223> primer annealing to third part #3 of coding region III

<400> 6
tggtctcagc cgccctat          18

**Description**

A method for providing a DNA-encoding library, the DNA-encoding library and a method of decoding a DNA-encoded library are presented. Many different DNA molecules are synthesized which differ from each other by comprising different DNA barcode sequences, wherein each DNA barcode sequence comprises at least a first coding region DNA sequence comprising at least a first part, a second part and a third part, wherein the second part is located between the first and third part and the second part differs between all the DNA molecules by at least two nucleotides. Each of the many different DNA molecules is bonded to at least a specific substance forming different DNA-substance conjugates, wherein the DNA-substance conjugates differ from each other by the specific substance and by their DNA molecules, wherein the first part and the third part encode information regarding the second part of the first coding region and wherein a certain first part and/or a certain third part uniquely codes for a certain group of DNA-substance conjugates which is smaller than the group of all DNA-substance conjugates in the DNA-encoded library. The DNA-encoded library has the advantage that, for example after an enrichment experiment performed with the library, the library may be decoded in a faster and less expensive manner than known DNA-encoded libraries.

In drug discovery which aims at identifying high affinity binders from a pool of molecules, it is known in the prior art to use a DNA-encoded library ("DEL"). Ideally, said DEL can mimic the function-information relationship of cells, such as T cells and B cells in adaptive immunity and peptide/protein-display technologies (e.g. phage display, ribosome display, yeast display). In T cells, B cells and/or phages, the functions (mediated e.g. by proteins expressed on cell surface) and associated information (coded e.g. by genetic information) are both confined in individual cells. The function-information relationship can be studied even if there is only a single copy of an individual cell presented in a given cell mixture.

A DEL is composed of a pool of different molecules, each being a conjugate between a small organic molecule and a specific DNA sequence (a so-called "DNA barcode"), thus realizing a direct physical connection between function (function of the small organic molecule by its chemical structure) and information (information about the type of small organic molecule coded by the DNA sequence). The DNA sequences are designed to identify the associated chemical structures using various technologies, e.g. Sanger sequencing, DNA array and/or high throughput sequencing.

Although PCR (polymerase chain reaction) is mainly used to amplify the selected compounds, PCR and real-time PCR (rtPCR) can also be used as a validation technique to check whether and at which abundance one particular DNA barcode is present, e.g. before and/or after the DEL has been subjected to a selection experiment. A selection experiment seeks to enrich certain conjugates between small organic molecules and DNA barcodes based on isolating said conjugates after they have bound to one or more desired target(s). Since the conjugates are enriched, a DEL selection experiment may be regarded as an experiment enriching certain DNA barcodes, namely those coding for small organic molecules having a high binding affinity to the target(s).

Similar like in the phage display technology, a usual DEL selection experiment provides tens to hundreds of DNA barcodes (DNA sequences) in one round of selection (one run). However, different from phage display technology, which regularly reveals organic molecules which are highly specific and potent binders (i.e. the $k_D$ to the target lies in the pM to nM range), a DEL selection experiment frequently also reveals DNA barcodes coding for small organic molecules which are only moderate binders (e.g. the $k_D$ to the target lies in the low to medium $\mu$M range).

In principle, Sanger sequencing provides a tool to decode DNA barcodes which have been found in a DEL selection experiment.

However, Sanger sequencing has the disadvantage that the throughput is low, i.e. the "reading" of the DNA barcode consumes a lot of time thus represents an uneconomical readout.

A further disadvantage of Sanger sequencing is its low sensitivity when analyzing complex mixtures of different DNA sequences. Assuming a DEL selection experiment using a DEL comprising 1 million different compounds, one compound is usually enriched 1000 times over the average and 100 sequences will be obtained from Sanger sequencing. In this case, there will be an approx. 90% chance that that one particular compound is not identified by the selection experiment, i.e. escapes identification, because its presence is not revealed by Sanger sequencing.

Moreover, even if a certain DNA barcode (e.g. coding for a certain small organic molecule) appears once in the enrichment process, Sanger sequence may identify said DNA barcode as coding for a small organic molecule which binds to the target. However, Sanger sequencing cannot reveal whether the identification of this specific small organic molecule has been a random event (i.e. an accidental hit) or is actually statistically significant (i.e. a true hit). In short, Sanger sequencing also suffers the disadvantage that false positives may not be distinguished from true positives without oversampling. While oversampling in the context of Sanger sequencing is apparently very important to obtain statistically meaningful results for hit identification in the decoding process (readout), it has become clear that Sanger sequencing is far from being efficient.

A DNA array provides an alternative solution to decode a DNA barcode sequence of binders identified in a DEL selection experiment. Since each DNA barcode sequence is associated with a certain physical location and evaluated according to its fluorescence intensity, the measurement avoids the requirement of oversampling using Sanger sequencing.

However, although fully complimentary sequences lead to highest signal intensity, strong background noise associated with mismatching DNA sequence interaction prevents the use of this method to decode a large library of DNA barcode sequences. For example, with a library of only few hundreds compounds each having a DNA barcode sequence, great effort needs to be made to distinguish a specific pair from mismatching and background noises. In short, the DNA array identification method also suffers the disadvantage that false positives may not be distinguished well from true positives. In other words, the systemic error of this identification method is high.

High throughput sequencing ("HTS") has become the standard technology for decoding a DEL after a selection experiment. HTS applies a similar principle like Sanger sequencing and uses the count of a particular sequence as an indicator of its enrichment. Millions of sequence reads resulting from HTS make oversampling possible, even when a DEL of a relatively large size is used.

However, like the DNA array approach, HTS can only provide a semiquantitative analysis of a selection experiment, because it was found that the counts of DNA barcode sequences and the measured affinity of its bound small organic molecule to the desired target(s) only show a poor correlation. The identified poor correlation has not yet been fully understood. Principally, it could be caused by a low synthetic quality of the DNA barcodes, while biases during the PCR and sequencing process may play a role. In summary, HTS is prone to reveal many false positive hits during the identification process, i.e. the systemic error of this identification method is high. Moreover, as the size of a DEL has been increasing gradually in recent years, HTS will no longer fulfill the requirement of oversampling when a DEL has started to comprise billions of compounds.

Furthermore, although HTS has become cheaper in the last years, it is still very expensive for many academic researchers. The outsourced sequencing tasks normally take a few weeks while researchers have no control over the sequencing experiments.

PCR and rtPCR have been used in the prior art to overcome the problems of the Sanger sequencing, DNA array and HTS identification methods. The advantages of both PCR and rtPCR are that primer pairs can be designed for a certain code. In other words, different primers may be used which themselves can carry a "code" in the sense that some of the primers bind (at least partially) to certain codes and some other do not. Additionally, rtPCR has the advantage over PCR that it will reveal a difference between a positive control and a negative control (in real time) and thus allows a better discrimination between false and true positives.

However, although rtPCR provides a quantitative analysis of a DEL selection process, it can only be designed for a limited number of codes and compounds. Therefore rtPCR suffers the disadvantage that it cannot be used for decoding the results of de novo selection experiments.

Starting herefrom, it was the object of the present invention to provide a method for encoding and decoding DNA barcodes having been enriched in a selection experiment with a DNA encoded library, wherein the method shall overcome the deficiencies of the prior art identification methods. Specifically, the method should be a facile, cost-efficient, quantitative, highly sensitive (i.e. be capable for revealing also weak binders), highly specific (i.e. be capable to reveal more true positives than false positives) and suitable to decode de novo selection experiments.

The object is solved by the method for providing a DNA-encoded library having the features of claim 1, the DNA-encoded library having the features of claim 5 and the method of decoding said DNA-encoded library having the features of claim 9. The subject-matter of the dependent claims illustrates advantageous embodiments of the invention.

According to the invention, a method for providing a DNA-encoded library (DEL) is provided, the method comprising

    a) synthesizing many different DNA molecules which differ from each other by comprising different DNA barcode sequences, wherein each DNA barcode sequence comprises at least a first coding region DNA sequence comprising at least a first part, a second part and a third part, wherein the second part is located between the first and third part and the second part differs between all the DNA molecules by at least two nucleotides; and

    b) bonding each of the many different DNA molecules to at least a specific substance forming different DNA-substance conjugates, wherein the DNA-substance conjugates differ from each other by the specific substance and by their DNA molecules;

characterized in that the first part and the third part encode information regarding the second part of the first coding region, wherein a certain first part and/or a certain third part uniquely codes for a certain group of DNA-substance conjugates which is smaller than the group of all DNA-substance conjugates in the DNA-encoded library.

The advantage of the DNA-encoded library ("DEL") provided by the inventive method is that both the first and third part of the DNA barcode sequence each encode for a certain subgroup of DNA-substance conjugates within the DEL. In qPCR, a primer binding to the first part of the DNA barcode sequence will give a strong signal (strong amplification) if the subgroup of DNA-substance conjugates for which the first part encodes (e.g. transcription factors) has been enriched in a previous selection experiment performed with the DEL. The same is true for the third part of the DNA barcode sequence, i.e. a primer binding to the third part of the DNA barcode sequence will give a strong signal (strong amplification) if the subgroup of DNA-substance conjugates for which the third part encodes (e.g. zinc finger proteins) has been enriched in a previous selection experiment performed with the DEL. If strong signal is obtained for both a primer binding to the first part and a primer binding to the third part after qPCR, the skilled person knows that DNA-substance conjugates belonging to both subgroups (e.g. zinc finger transcription factors) have been strongly enriched. The skilled person obtains this information only via qPCR with the inventive DEL and suitable primers, i.e. the skilled person does not have to perform a DNA sequencing. This allows a much faster and less expensive decoding of a DNA-encoded library after a selection experiment performed with said library.

The DNA-encoded library can be used to construct many two-dimensional matrices in which different first primers which bind to different first parts of the barcode form the rows of the matrix, different second primers which bind to different second parts of the barcode are the columns of the matrix and the signal intensity after qPCR with each primer pair is given in each each field of the matrix (crossing point between rows and colums). The signal intensity obtained for each primer pairing allows a deconvolution of the mixture of DNA barcodes, i.e. of the DEL after the selection experiment. The possibility to deconvolute the mixture of DNA barcodes strongly improves the specificity of the identification method, i.e. its capability of distinguishing true positive hits from false positive hits and allows a quick determination of "hits" even without performing DNA sequencing.

Since performing qPCR without DNA sequencing is not expensive, it is estimated that a full decoding experiment will cost only approx. 50 €. Thus, the DEL produced with the inventive method allows a very cost-efficient "hit" detection after an enrichment experiment with said DEL and needs very little invenstment in instrumentation. Additionally, the DEL allows to obtain a more quantitative information on the abundance of a certain DNA barcodes after a selection experiment as compared with previously known DELs.

The inventive method can be characterized in that

i) the first coding region DNA sequence comprises at least a fourth part, wherein the second part is located between the fourth and third part and wherein both the combination of the first part and the fourth part and the combination of the first part and the third part of the first coding region encode information about the second part of the first coding region; and

ii) each barcode sequence comprises at least a second coding region DNA sequence comprising at least a first part, a second part, a third part, and a fourth part, wherein the second part is located between the fourth and third part and the second part differs between all the DNA molecules by at least two nucleotides, wherein both the combination of the first part and the fourth part and the combination of the first part and the third part of the second coding region encode information about the second part of the second coding region;

wherein a certain combination of a first part and fourth part in a certain coding region uniquely codes for a certain group of DNA-substance conjugates which is smaller than the group of all DNA-substance conjugates which is encoded by the first part alone.

In this embodiment of the invention, the DNA-encoded library can be used to construct more two-dimensional matrices because an additional primer can be used which anneals to the fourth part of the DNA barcode and because a further coding region with different four parts is present. Only with one single run of qPCR, very detailed information is obtained about the specific groups of DNA-substance conjugates that have been enriched in the selection experiment with the DEL.

Furthermore, the inventive method can be characterized in that

i) each barcode sequence comprises at least a second coding region DNA sequence comprising at least a first part, a second part, a third part, and a fourth part, wherein the second part is located between the fourth and third part and the second part differs between all the DNA molecules by at least two nucleotides, wherein both the combination of the first part and the fourth part and the combination of the first part and the third part of the second coding region encode information about the second part of the second coding region; and

ii) each barcode sequence comprises at least a third coding region DNA sequence comprising at least a first part, a second part, a third part, and a fourth part, wherein the second part is located between the fourth and third part and the second part differs between all the DNA molecules by at least two nucleotides, wherein both the combination of the first part and the fourth part and the combination of the first part and third part and the of the third coding region encode information about the second part of the third coding region;

wherein a certain combination of a first part and fourth part in a certain coding region uniquely codes for a certain group of DNA-substance conjugates which is smaller than the group of DNA-substance conjugates which is encoded by the first part.

In view of the further coding region and the separation of at least one coding region into five parts, more different primers can be used in one single qPCR and within one single run of qPCR, very detailed information can obtained which specific groups of DNA-substance conjugates have been enriched in the selection experiment with the DEL.

In a preferred embodiment of the invention, at least one coding region DNA sequence, optionally all coding region DNA sequences, comprise at least a first part, a second part, a third part, a fourth part and a fifth part, wherein the second part is located between the fourth and fifth part and the second part differs between all the DNA molecules by at least two nucleotides, wherein the combination of the first part and the fourth part and the combination of the fifth part and the third part of the coding region encode information about the second part of the coding region, preferably of all coding regions, wherein a certain combination of a first part and fourth part uniquely codes for a certain group of DNA-substance conjugates which is smaller than the group of DNA-substance conjugates which is encoded by the first part alone, and wherein a certain combination of a fifth part and third part uniquely codes for a certain group of DNA-substance conjugates which is smaller than the group of DNA-substance conjugates which is encoded by the third part alone.

Since in this embodiment, at least one coding region has not three or four, but actually five parts, a total of four primers can be used in each qPCR for amplifying the at least one coding region. In short, one primer annealing to the first part, one primer annealing to the third part, one primer annealing to the fourth part and one primer annealing to the fifth part can be used. This gives a total amount of 6 two-dimensional matrices. Thus, in one single qPCR, more detailed information is obtained which specific groups of DNA-substance conjugates have been enriched in the selection experiment with the DEL.

Furthermore, according to the invention, a DNA-encoded library is provided. The DNA-encoded library comprises many different DNA-substance conjugates, wherein the DNA-substance conjugates differ from each other by their substance and by their DNA molecules, wherein the DNA molecules of the DNA-substance conjugates differ from each other by comprising different DNA barcode sequences, wherein each DNA barcode sequence comprises at least a first coding region DNA sequence comprising at least a first part, a second part and a third part, wherein the second part is located between the first and third part and the second part differs between all the DNA molecules by at least two nucleotides, characterized in that the first part and the third part encode information regarding the second part of the first coding region, wherein a certain first part and/or a certain third part uniquely codes for a certain group of DNA-substance conjugates which is smaller than the group of all DNA-substance conjugates in the DNA-encoded library.

The inventive DNA-encoded library can be characterized in that

i) the first coding region DNA sequence comprises at least a fourth part, wherein the second part is located between the fourth and third part and wherein both the combination of the first part and the fourth part and the combination of the first part and the third part of the first coding region encode information about the second part of the first coding region; and

ii) each barcode sequence comprises at least a second coding region DNA sequence comprising at least a first part, a second part, a third part, and a fourth part, wherein the second part is located between the fourth and third part and the second part differs between all the DNA molecules by at least two nucleotides, wherein both the combination of the first part and the fourth part and the combination of the first part and the third part of the second coding region encode information about the second part of the second coding region;

wherein a certain combination of a first part and fourth part in a certain coding region uniquely codes for a certain group of DNA-substance conjugates which is smaller than the group of all DNA-substance conjugates which is encoded by the first part alone.

Furthermore, the inventive DNA-encoded library can be characterized in that ach barcode sequence comprises at least a third coding region DNA sequence, which is on the same DNA strand as the second coding region, comprising at least a first part, a second part, a third part, and a fourth part, wherein the second part is located between the fourth and third part and the second part differs between all the DNA molecules by at least two nucleotides, wherein both the combination of the first part and the fourth part and the combination of the first part and the third part and the of the third coding region encode information about the second part of the third coding region, wherein a certain combination of a first part and fourth part in the second coding region and in the third coding region uniquely codes for a certain group of DNA-substance conjugates which is smaller than the group of DNA-substance conjugates which is encoded by the first part alone.

In a preferred embodiment of the invention, the DNA-encoded library is characterized in that at least one coding region DNA sequence, optionally all coding region DNA sequences, comprise at least a first part, a second part, a third part, a fourth part and a fifth part, wherein the second part is located between the fourth and fifth part and the second part differs between all the DNA molecules by at least two nucleotides, wherein the combination of the first part and the fourth part and the combination of the fifth part and the third part of the coding region encode information about the second part of the coding region, preferably of all coding regions, and wherein a certain combination of a first part and fourth part uniquely codes for a certain group of DNA-substance conjugates which is smaller than the group of DNA-substance conjugates which is encoded by the first part alone and wherein a certain combination of a fifth part and third part uniquely codes for a certain group of DNA-substance conjugates which is smaller than the group of DNA-substance conjugates which is encoded by the third part alone.

In a further preferred embodiment, the DNA-encoded library is producible or produced by the inventive method for providing a DNA-encoded library,

Moreover, according to the invention, a method of decoding the inventive DNA-encoded library is provided. The method comprises

a) performing a qPCR with the DNA-encoded library according to one of claims 5 to 8 as template, wherein the following primers are used:

a primer A and a primer B for amplifying the first coding region of every DNA-substance conjugate; and many different primers A-xN which anneal to the different first parts of the first coding region and many different primers B-yN which anneal to the different third parts of the first coding region, wherein primer A-xN has an identical length like the coding region primer A by shortening x nucleotides at its 5'-end, primer B-yN has an identical length like the coding region primer B by shortening y nucleotides at its 5'-end, N represents a A, T, G or C and x and y represent the total number of any one of A, T, G or C at the 3'-end of the primers, wherein x is an integer from 2 to 6, preferably 4;

b) calculating a mathematical product of the signal value of each primer A-xN and each primer B-xN by following equation:

$$\text{Value } (A\text{-}xN)_i = \text{signal value } [(A\text{-}xN)_i + B] \cdot \text{signal value } [(A\text{-}xN)_i + (B\text{-}xN)_i];$$

and

$$\text{Value } (B\text{-}yN)_i = \text{signal value } [(B\text{-}yN)_i + A] \cdot \text{signal value } [(B\text{-}yN)_i + (A\text{-}xn)_i],$$

wherein i is an integer and defines a specific primer, and the "+"-sign indicates a combination of two primers; wherein signal value is the percentage of abundance related to the whole set of qPCR quantification using different primers annealed to the same region; and

c) comparing the obtained mathematical products for each of the primers $(A\text{-}xN)_i$ and $(B\text{-}yN)_i$, wherein those primers with high values code for DNA-substance conjugates which are present at a high concentration in the DNA-encoded library.

The method of decoding the inventive DNA-encoded library can be characterized in that the method comprises

i) calculating a mathematical product of the value obtained for each primer A-xN and each primer B-yN by following equation

$$\text{Value } (A\text{-}B)_i = \text{Value } (A\text{-}xN)_i \cdot \text{Value } (B\text{-}yN)_i;$$

ii) comparing the obtained mathematical products for each of the combination of primers $(A\text{-}xN)_i$ and $(B\text{-}yN)_i$, wherein those primer combinations with high values code for DNA-substance conjugates which are present at a high concentration in the DNA-encoded library.

Furthermore, the method of decoding the inventive DNA-encoded library can be characterized in that the qPCR is performed with the inventive DNA-encoded library according and the method comprises

i) performing a qPCR with the following primers:

a first coding region primer A and a first coding region primer primer B for amplifying the first coding region of every DNA-substance conjugate; and many different primers A-xN which anneal to the different first parts, or first and fourth parts of the first coding region and many different primers B-yN which anneal to the different third parts of the first coding region, wherein A-xN has an identical length like the coding region primer A by shortening x nucleotides at its 5'-end, B-yN has an identical length like the coding region primer B by shortening y nucleotides at its 5'-end, N represents a A, T, G or C and x and y represent the total number of any one of A, T, G or C at the 3'-end of the primers, wherein x is an integer from 6 to 10, preferably 8, and y is an integer from 2 to 6, preferably 4; and

a second coding region primer C and a second coding region primer D for amplifying the second coding region of every DNA-substance conjugate; and

many different primers D-yN which anneal to the different first parts, or first and fourth parts of the second coding region and many different primers C-xN which anneal to the different third parts of the second coding region, wherein primer C-xN has an identical length like the coding region primer C by shortening x nucleotides at its 5'-end, primer D-yN has an identical length like the coding region primer D by shortening y nucleotides at its 5'-end, N represents a A, T, G or C and x and y represent the total number of any one of A, T, G or C at the 3'-end of the primers, wherein x is an integer from 6 to 10, preferably 8, and y is an integer from 2 to 6, preferably 4;

ii) calculating a mathematical product of the signal value of each primer A-xN, each primer B-yN, each primer C-xN and each primer D-yN by following equation:

$$\text{Value } (A\text{-}xN)_i = \text{signal value } [(A\text{-}xN)_i + B] \cdot \text{signal value } [(A\text{-}xN)_i + (B\text{-}xN)_i];$$

$$\text{Value } (B\text{-}yN)_i = \text{signal value } [(B\text{-}yN)_i + A] \cdot \text{signal value } [(B\text{-}yN)_i + (A\text{-}xN)_i],$$

$$\text{Value } (C\text{-}xN)_i = \text{signal value } [(C\text{-}xN)_i + D] \cdot \text{signal value } [(C\text{-}xN)_i + (D\text{-}xn)_i],$$

$$\text{Value } (D\text{-}yN)_i = \text{signal value } [(D\text{-}yN)_i + C] \cdot \text{signal value } [(D\text{-}yN)_i + (C\text{-}xn)_i],$$

wherein i is an integer and defines a specific primer, and the "+"-sign indicates a combination of two primers; wherein signal value is the percentage of abundance related to the whole set of qPCR quantification using different primers annealed to the same region; and

iii) comparing the obtained mathematical products for each of the primers $(A\text{-}xN)_i$, $(B\text{-}yN)_i$, $(C\text{-}xN)_i$ and $(D\text{-}yN)_i$, wherein those primers with high values code for DNA-substance conjugates which are present at a high concentration in the DNA-encoded library.

In a preferred embodiment of the invention, the method of decoding the inventive DNA-encoded library comprises

i) calculating a mathematical product of the value obtained for each primer A-xN and each primer B-yN, for each primer A-xN and each primer D-xN and for each primer C-yN and D-xN by following equation

$$\text{Value } (A\text{-}B)_i = \text{Value } (A\text{-}xN)_i \cdot \text{Value } (B\text{-}yN)_i;$$

$$\text{Value } (A\text{-}D)_i = \text{Value } (A\text{-}xN)_i \cdot \text{Value } (D\text{-}yN)_i;$$

$$\text{Value } (C\text{-}D)_i = \text{Value } (C\text{-}xN)_i \cdot \text{Value } (D\text{-}yN)_i;$$

ii) calculating the mathematical product of the Value $(A\text{-}B)_i$, $(A\text{-}D)_i$ and $(C\text{-}D)_i$ for each primer i by the following equation

$$\text{Value}^i = \text{value } (A\text{-}B)_i \cdot \text{value } (A\text{-}D)_i \cdot \text{value } (C\text{-}D)_i$$

iii) comparing the obtained mathematical products Value$^i$, wherein those primer combinations i with high values code for DNA-substance conjugates which are present at a high concentration in the DNA-encoded library.

In a further preferred embodiment, the method of decoding the inventive DNA-encoded library is characterized in that the qPCR is performed with the inventive DNA-encoded library as template and the method comprises

i) performing a qPCR with the following primers:

a first coding region primer A and a first coding region primer B for amplifying the first coding region of every DNA-substance conjugate; and many different primers A-xN which anneal to the different first parts, or first and fourth parts of the first coding region, and many different primers B-yN which anneal to the different third parts of the first coding region, wherein A-xN has an identical length like the coding region primer A by shortening x nucleotides at its 5'-end, B has an identical length like the coding region primer B-yN by shortening y nucleotides at its 5'-end, N represents a A, T, G or C and x and y represent the total number of any one of A, T, G or C at the 3'-end of the primers, wherein x is an integer from 6 to 10, preferably 8, and y is an integer from 2 to 6, preferably 4; and
a second coding region primer C and a second coding region primer D for amplifying the second coding region of every DNA-substance conjugate; and
many different primers D-yN which anneal to the different first parts, or first and fourth parts of the second coding region and many different primers C-xN which anneal to the different third parts of the second coding region, wherein primer C-xN has an identical length like the coding region primer C by shortening x nucleotides at its 5'-end, primer D-yN has an identical length like the coding region primer D by shortening y nucleotides at its 5'-end, N represents a A, T, G or C and x and y represent the total number of any one of A, T, G or C at the 3'-end of the primers, wherein x is an integer from 6 to 10, preferably 8, and y is an integer from 2 to 6, preferably 4;
a third coding region primer E and a third coding region primer F for amplifying the third coding region of every DNA-substance conjugate; and many different primers E-xN which anneal to the different first parts of the third coding region and many different primers F-yN which anneal to the different third parts of the third coding region, wherein primer E-xN has an identical length like the coding region primer E by shortening x nucleotides at its 5'-end, primer F-yN has an identical length like the coding region primer F by shortening y nucleotides at its 5'-end, N represents a A, T, G or C and x and y represent the total number of any one of A, T, G or C at the 3'-end of the primers, wherein x is an integer from 6 to 10, preferably 8, and y is an integer from 2 to 6, preferably 4;

ii) calculating a mathematical product of the signal value of each primer A-xN, each primer B-yN, each primer C-xN, each primer D-yN, each primer E-xN and each primer F-yN by following equation:

$$\text{Value } (A\text{-}xN)_i = \text{signal value } [(A\text{-}xN)_i + B] \cdot \text{signal value } [(A\text{-}xN)_i + (B\text{-}xN)_i];$$

$$\text{Value } (B\text{-}yN)_i = \text{signal value } [(B\text{-}yN)_i + A] \cdot \text{signal value } [(B\text{-}yN)_i + (A\text{-}xN)_i],$$

$$\text{Value } (C\text{-}xN)_i = \text{signal value } [(C\text{-}xN)_i + D] \cdot \text{signal value } [(C\text{-}xN)_i + (D\text{-}xN)_i],$$

$$\text{Value } (D\text{-}yN)_i = \text{signal value } [(D\text{-}yN)_i + C] \cdot \text{signal value } [(D\text{-}yN)_i + (C\text{-}xN)_i],$$

$$\text{Value } (E\text{-}xN)_i = \text{signal value } [(E\text{-}xN)_i + F] \cdot \text{signal value } [(E\text{-}xN)_i + (F\text{-}xn)_i],$$

Value $(F\text{-}yN)_i$ = signal value $[(F\text{-}yN)_i + E] \cdot$ signal value $[(F\text{-}yN)_i + (E\text{-}xN)_i]$, wherein i is an integer and defines a specific primer, and the "+"-sign indicates a combination of two primers; wherein signal value is the percentage of abundance related to the whole set of qPCR quantification using different primers annealed to the same region; and
iii) comparing the obtained mathematical products for each of the primers $(A\text{-}xN)_i$, $(B\text{-}yN)_i$, $(C\text{-}xN)_i$, $(D\text{-}yN)_i$, $(E\text{-}xN)_i$ and $(N\text{-}yN)_i$, wherein those primers with high values code for DNA-substance conjugates which are present at a high concentration in the DNA-encoded library.

The method of decoding the inventive DNA-encoded library may comprise

i) calculating a mathematical product of the value obtained for each primer A-xN and each primer B-yN, for each primer A-xN and each primer D-xN, for each primer C-yN and D-xN, for each primer A-xN and N-yN, for each primer M-xN and D-yN and for each primer M-xN and N-yN by following equation

$$\text{Value } (A\text{-}B)_i = \text{Value } (A\text{-}xN)_i \cdot \text{Value } (B\text{-}yN)_i;$$

$$\text{Value } (A\text{-}D)_i = \text{Value } (A\text{-}xN)_i \cdot \text{Value } (D\text{-}yN)_i;$$

$$\text{Value } (C\text{-}D)_i = \text{Value } (C\text{-}xN)_i \cdot \text{Value } (D\text{-}yN)_i;$$

$$\text{Value } (A\text{-}F)_i = \text{Value } (A\text{-}xN)_i \cdot \text{Value } (F\text{-}yN)_i;$$

$$\text{Value } (E\text{-}D)_i = \text{Value } (E\text{-}xN)_i \cdot \text{Value } (D\text{-}yN)_i;$$

$$\text{Value } (E\text{-}F)_i = \text{Value } (E\text{-}xN)_i \cdot \text{Value } (F\text{-}yN)_i;$$

ii) calculating the mathematical product of the values $(A\text{-}B)_i$, $(A\text{-}D)_i$, $(C\text{-}D)_i$, $(A\text{-}F)_i$, $(E\text{-}D)_i$ and $(E\text{-}F)_i$ for each primer combinations i by the following equation

$$\text{Value}^i = \text{value } (A\text{-}B)_i \cdot \text{value } (A\text{-}D)_i \cdot \text{value } (C\text{-}D)_i \cdot \text{value } (A\text{-}F)_i \cdot \text{value } (E\text{-}D)_i \cdot \text{value } (E\text{-}F)_i;$$

iii) comparing the obtained mathematical products Value$^i$, wherein those primer combinations i with high values code for DNA-substance conjugates which are present at a high concentration in the DNA-encoded library.

In a preferred embodiment, the method is characterized in that it comprises the calculation of a Value$^i$ by the following calculation:

$$\text{Value}^{i'} = \log_{10}[\text{value } (A\text{-}B)_i \cdot \text{value } (A\text{-}D)_i \cdot \text{value } (C\text{-}D)_i \cdot \text{value } (A\text{-}F)_i \cdot \text{value } (E\text{-}D)_i \cdot \text{value } (E\text{-}F)_i].$$

With reference to the following Figures and Examples, the subject according to the invention is intended to be explained in more detail without wishing to restrict said subject to the special embodiments shown here.

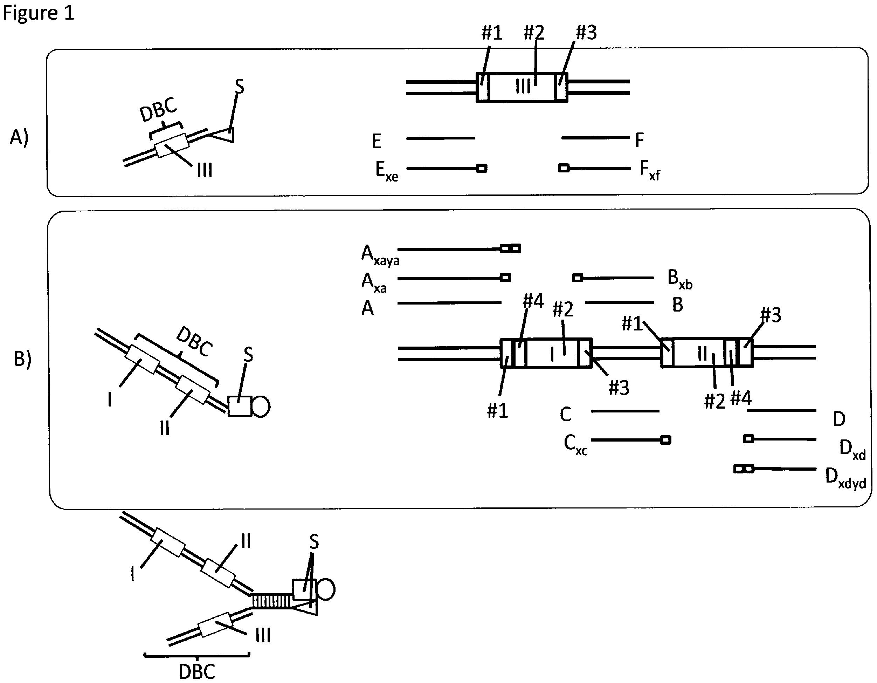

Figure 1A shows how the coding algorithm works for generating a qPCR-matrix for DNA codes 1 having one single coding region 2 (coding region III). E and F are primary primers and $E_{xe}$ and $F_{xf}$ are secondary primers. Primary primer E binds upstream (i.e. towards the 5'-end) of the first region #1 and primary primer F binds upstream (i.e. towards the 5'-end) of the third region #3. A qPCR with only the two primary primers E, F amplifies the DNA barcodes of all DNA-substance conjugates of the DNA-encoded library having coding region III. A qPCR with at least one primary primer E, F and at least one secondary primer $E_{xe}$, $F_{xf}$ is called a "primary qPCR". Figure 1A illustrates a qPCR template containing one single coding region III having the three code parts (sub-codes) #1, #2, #3. The sequence of the second part #2 of the coding region III is a unique sub-code. Each combination of the first part #1 and the third part #3 can also represent a unique code. Therefore, a sequence of the second part #2 is corresponding to a combination of the first part #1 and the third part #3. For each code part (sub-code) #1, #2, #3, there is a minimal difference number n between any pair of sequences (e.g. between two different xe sequences), while n should be $\geq 2$. This means that the code parts #1, #2, #3 differs from each other by at least two nucleotides.

Figure 1B shows how the coding algorithm works for generating a qPCR-matrix for DNA codes having two coding regions, namely coding region I and coding region II. A, B, C and D are primary primers, $A_{xa}$, $B_{xb}$, $C_{xc}$ and $D_{xd}$ are secondary primers and $A_{xaya}$ and $D_{xdyd}$ are tertiary primers. A qPCR comprising the use of at least two tertiary primers is called a "tertiary PCR". Figure 1B illustrates a qPCR template containing two different coding regions I, II, wherein the first coding region I has four code parts (sub-codes) #1, #2, #3, #4 and the second coding region II also has four code parts (sub-codes) #1, #2, #3, #4. The sequence of the second parts #2 of each coding region I, II represents a unique sub-code. Each combination of the first part #1 and the third part #3 of each coding region I, II can also represent a unique sub-code of each coding region I, II. In this case, a sequence of the second part #2 of each coding region I, II is corresponding to a combination of the sequence of the first part #1 and the sequence of the third part #3 of each coding region I, II. Each combination of the first part #1 the fourth part #4 can also represent one unique building block. In this case, a sequence of the second part #2 of each coding region I, II is also corresponding to a combination of the sequence of the first part #1 and the sequence of the fourth part #4 of each coding region I, II. For each code part (sub-code) #1, #2, #3, #4, there is a minimal difference number n between any pair of sequences (e.g. between two different #2 sequences), while n should be $\geq$ 2. This means that each code part #1, #2, #3, #4 differs from another code part #1, #2, #3, #4 by at least two nucleotides.

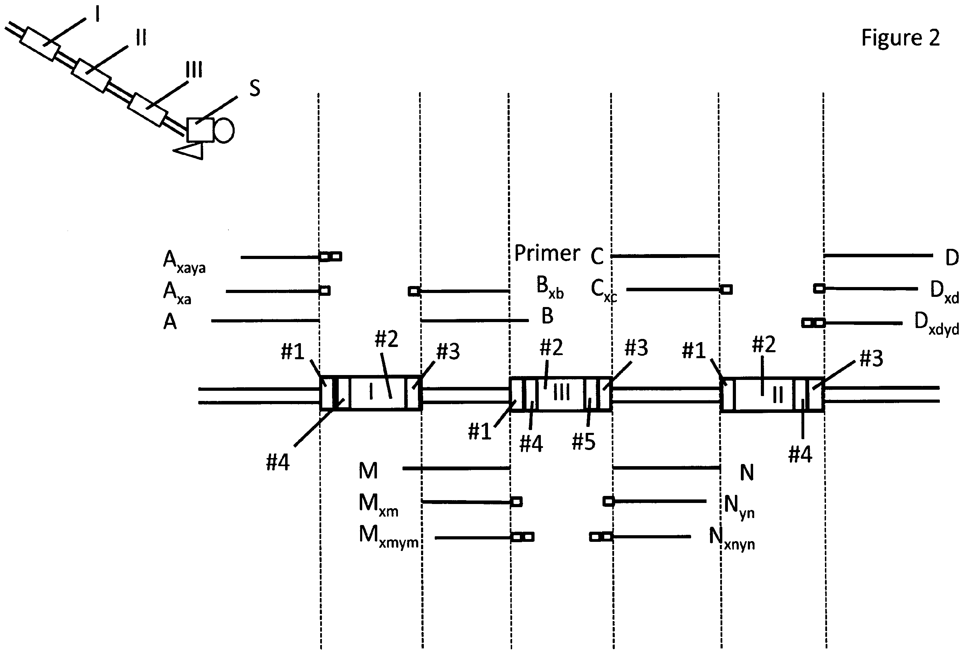

Figure 2 shows how the coding algorithm works for generating a qPCR-matrix for DNA codes having three coding regions I, II, III. A, B, C and D are each a primary primer. $A_{xa}$, $B_{xb}$, $C_{xc}$, $D_{xd}$, $M_{xm}$ and $N_{xn}$ are each a secondary primer. $A_{xaya}$, $D_{xdyd}$, $M_{xmym}$ and $N_{xnyn}$ are each a tertiary primer. A qPCR using at least two tertiary primers is called a "tertiary PCR". Figure 2 illustrates a qPCR template containing three different coding regions I, II, III, wherein the first coding region I has four code parts (sub-codes) #1, #2, #3, #4, the second coding region II also has four code parts (sub-codes) #1, #2, #3, #4 and the third coding region III has five code parts (sub-codes) #1, #2, #3, #4, #5. The sequence of each second code part #2 of each coding region I, II, III is a unique sub-code. Each combination of code parts #1 and code part #3, code part #1 with code part #4 and code part #1 with code part #5 can also represent a unique sub-code. For example, a sequence of code part #2 is corresponding to a combination of code part #1 and code part #3. For each code part (sub-code), there is a minimal difference number n between any pair of sequences (e.g. between two different ab sequences), while n should be $\geq$ 2. This means that each code part #1, #2, #3, #4, #5 differs from another code part #1, #2, #3, #4, #5 by at least two nucleotides.

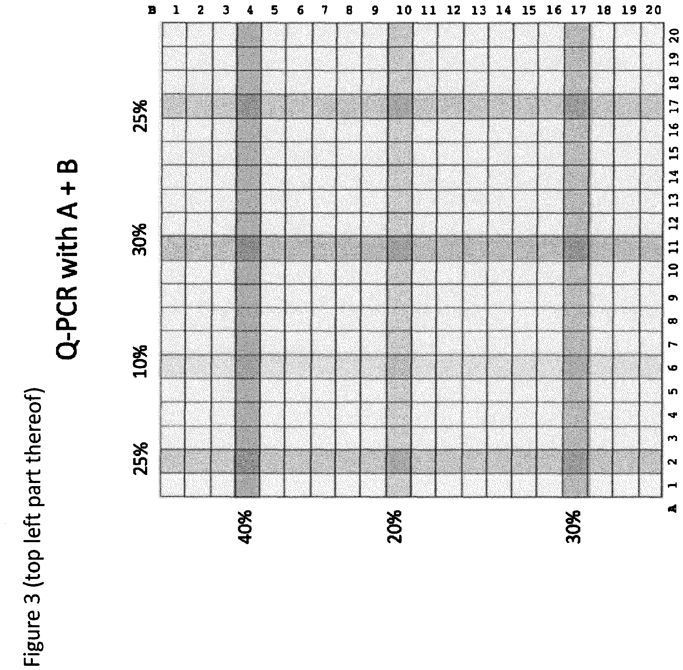

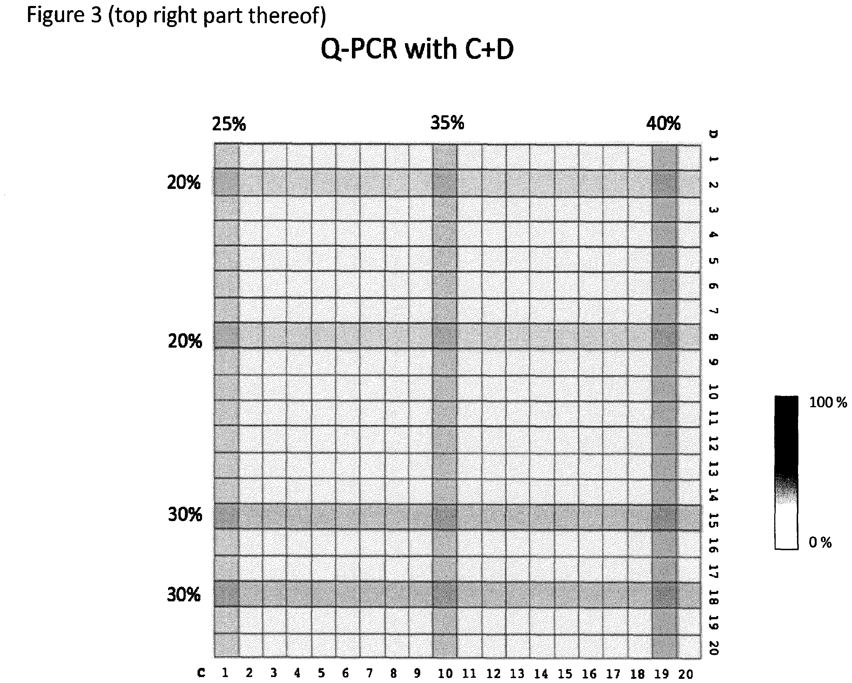

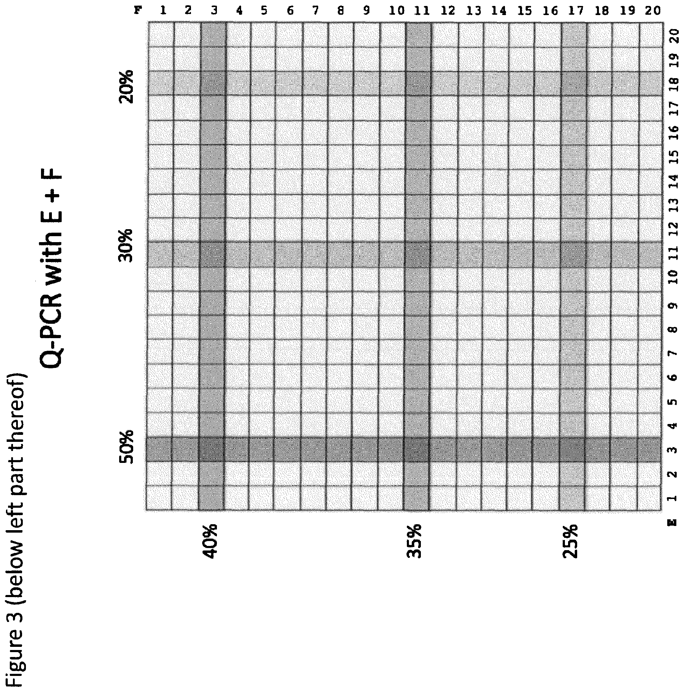

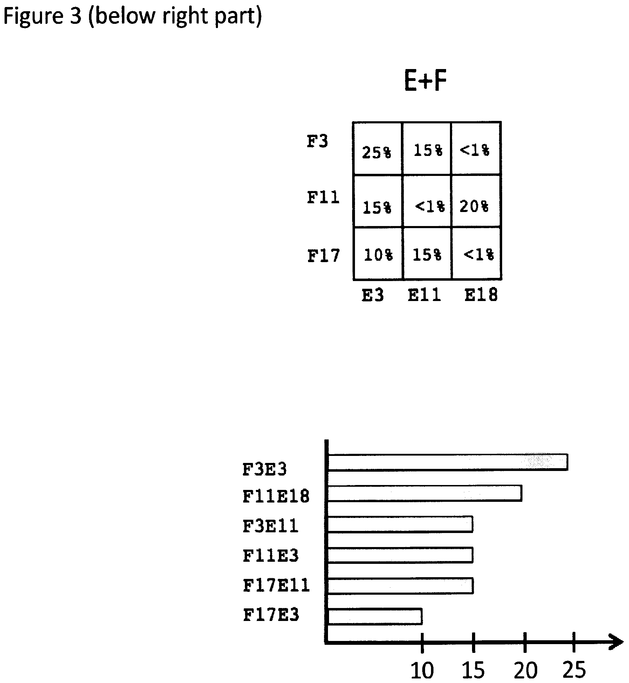

Figure 3 shows three different qPCR matrices which were obtained after a qPCR using a DNA barcode having three coding regions I, II, III as template and 20 different primary primers A and 20 different primary primers B for binding to coding region I (see matrix "Q-PCR with A + B", columns = different primers A, lines = different primers B), 20 different primary primers C and 20 different primary primers D for coding region II (see matrix "Q-PCR with C + D", columns = different primers C, lines = different primers D) and 20 different primary primers E and 20 different primary primers F for coding region III (see matrox "Q-PCR with E + F", columns = different primers E, lines = different primers F). An exemplary result of the matrix is illustrated in the table "E+F" in which the primer pairs with the strongest amplification signal are listed together with their obtained (normalized) amplification signal. It can be derived from said table that the strong amplification signal have been obtained with the primer pairs E3 and F3 (25%), E18 and F11 (20%), E3 and F11 (15%), E11 and F3 (15%) and E11 and F17 (15%) and a medium amplification signal has been obtained with the primer pair E3 and F17 (10%). Below the table "E+F", the obtained result is also shown in a column diagram. It can be derived from the obtained result that DNA-substance conjugates to which e.g. the primer pair E3 and F3 binds had a high concentration in the DNA-encoded library (after the enrichment experiment) and DNA-substance conjugates to which e.g. the primer pair E3 and F17 binds had a lower concentration in the DNA-encoded library (after the enrichment experiment). It may also be concluded that DNA-substance conjugates to which primer pairs with no signal (e.g. E1 and F1) bind were not present in the DNA-encoded library (after the enrichment experiment). Since the substance connected to each specific DNA coding region is known, this approach allows a fast and sensitive identification of substances being present at a high concentration after a (DEL) selection experiment.

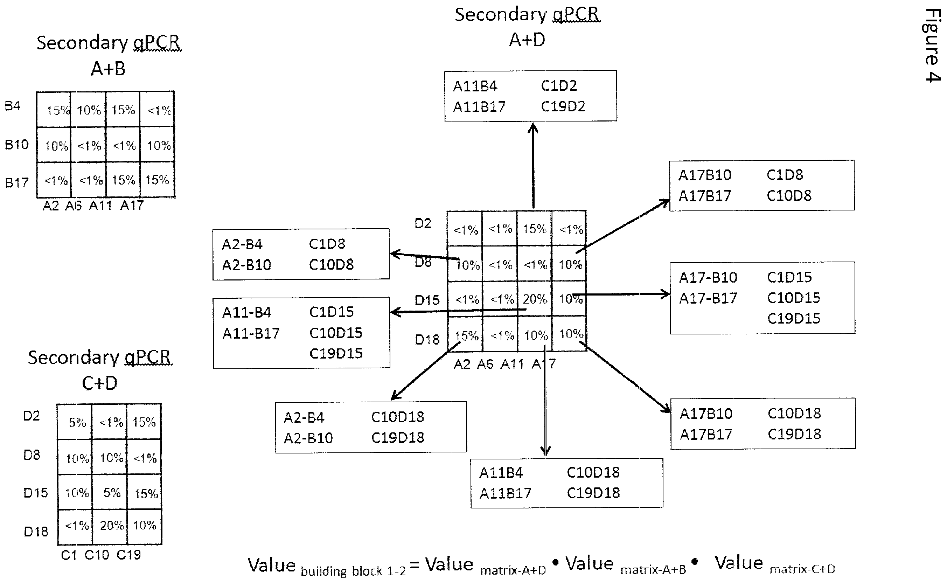

Figure 4 shows the results of the qPCR matrices "Q-PCR with A + B" (amplification of coding region I) and "Q-PCR with C + D" (amplification of coding region II) from Figure 3 and also the results of a secondary PCR with primer pairs A and D (coding region between I and II; see Figure 2). qPCR with the primer pair A + D gave strong amplification signals for the specific primer pairs A11 and D15 (20%), A11 and D2 (15%), A2 and D18 (15%) and medium amplification signals for the primer pairs A2 and D8 (10%), A11 and D18 (10%), A17 and D18 (10%), A17 and D15 (10%) and A17 and D8 (10%). Each of said identified A primers binds to a specific coding region I and each of said identified D primers binds to a specific coding region II. This means that the primers A and D which gave strong signals code for coding regions I and II which must have been enriched in the DNA-encoded library after the enrichment experiment. It can also be concluded that the two coding regions I and II must be located on one single DNA strand because otherwise, no amplification signal would have been obtained. In order to combine the result obtained with the primer pair A and D with the result of the other primer pairs A and B and C and D, the mathematical product of the value obtained for each specific primer pair is calculated by the equation $\text{Value}_{\text{coding region I-II}} = \text{Value}_{\text{matrix-A+D}} \cdot \text{Value}_{\text{matrix-A+B}} \cdot \text{Value}_{\text{matrix-C+D}}$. Specific primers A, B, C and D which resulted in a high amplification signal consequently have a high $\text{Value}_{\text{coding region I-II}}$. Thus, the obtained $\text{Value}_{\text{coding region I-II}}$ allows the identification of primers which must have bound to abundant DNA-barcodes and thus allows the identification of substances (bound to the DNA-barcode) which were abundantly present after the (DEL) selection experiment.

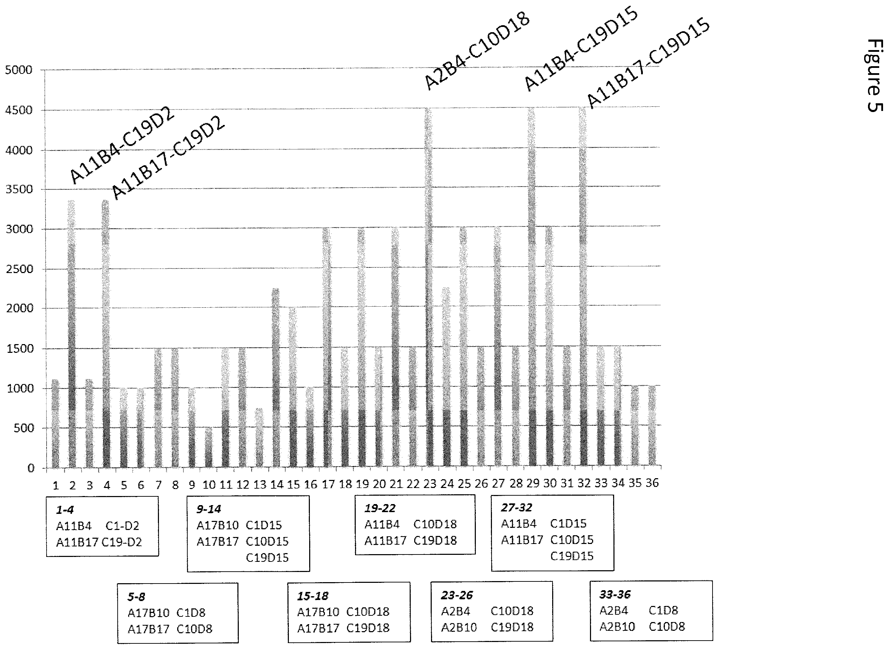

Figure 5 shows a plot of 36 different combinations of coding regions I (A + B) and II (C + D) which gave the highest mathematical product according to equation $\text{Value}_{\text{coding region I-II}} = \text{Value}_{\text{matrix-A+D}} \cdot \text{Value}_{\text{matrix-A+B}} \cdot \text{Value}_{\text{matrix-C+D}}$ (see absolute value in arbitrary unit on y-axis). In said plot, it can be visually identified that the combinations of coding region I and II with the numbers 2, 4, 23, 29 and 32 on the x-axis achieved the highest score. These numbers refer to the following five different combinations of coding regions I and II: A11B4-C19D2 (no. 2), A11B17-C19D2 (no. 4), A2B4-C10-D18 (no. 23), A11B4-C19D15 (no. 29) and A11B17-C19D15 (no. 32). Naturally, it is known for which substances (or plurality of substances) these five different combinations encode. Thus, it is possible to identify five different (groups of) substances which have been strongly enriched in a (DEL) selection experiment.

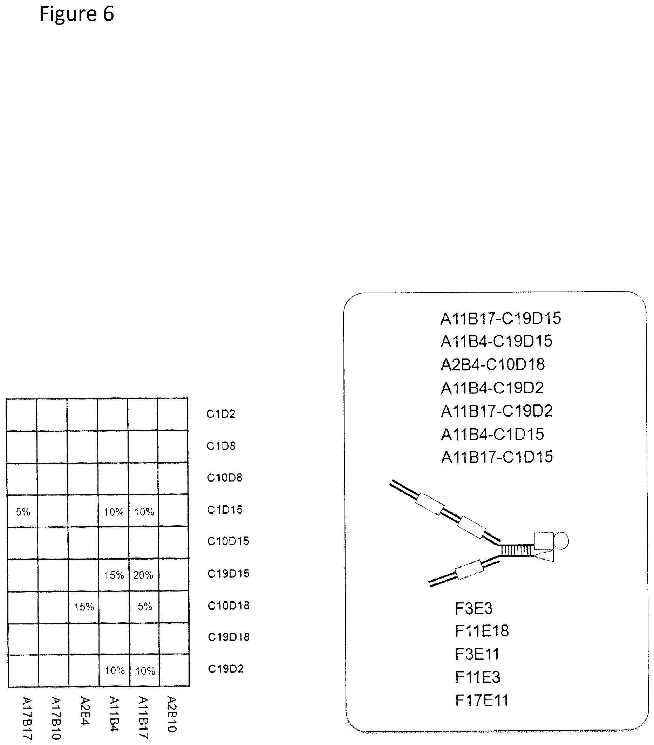

Figure 6 shows a part of qPCR matrices obtained after a qPCR using a DNA barcode having three coding regions I, II, III as template, 20 different primary primers A and B for coding region I, 20 different primary primers C and D for coding region II, the (same) 20 different primary primers A and D for coding region I to II and 20 different primary primers E and F for coding region III. After having caluculated the $\text{Value}_{\text{coding region I-II}} = \text{Value}_{\text{matrix-A+D}} \cdot \text{Value}_{\text{matrix-A+B}} \cdot \text{Value}_{\text{matrix-C+D}}$, it has become clear that significant values are obtained for coding regions I-II coded by the nine primer pairings A17B17-C1D15, A2B4-C10D18, A11B4-C1D15, A11B4-C19D15, A11B4-C19D2, A11B17-C1D15, A11B17-C19D15, A11B17-C10D18 and A11B17-C19D2. The highest value for coding region III has been determined as well by equation $\text{Value}_{\text{coding region III}} = \text{Value}_{\text{matrix-F+E}}$ and it has been found that high values for coding region III are obtained by the five primer pairings F3E3, F11E18, F3E11, F11E3 and F17E11. If the nine coding regions I to II identified above encode a first group of nine different substances and the five coding regions III identified above encode a second group of five different substances, it follows that the combination of the substances of the first group and second group must have been present at a high concentration before the qPCR experiment, i.e. must have been strongly enriched by the (DEL) selection experiment.

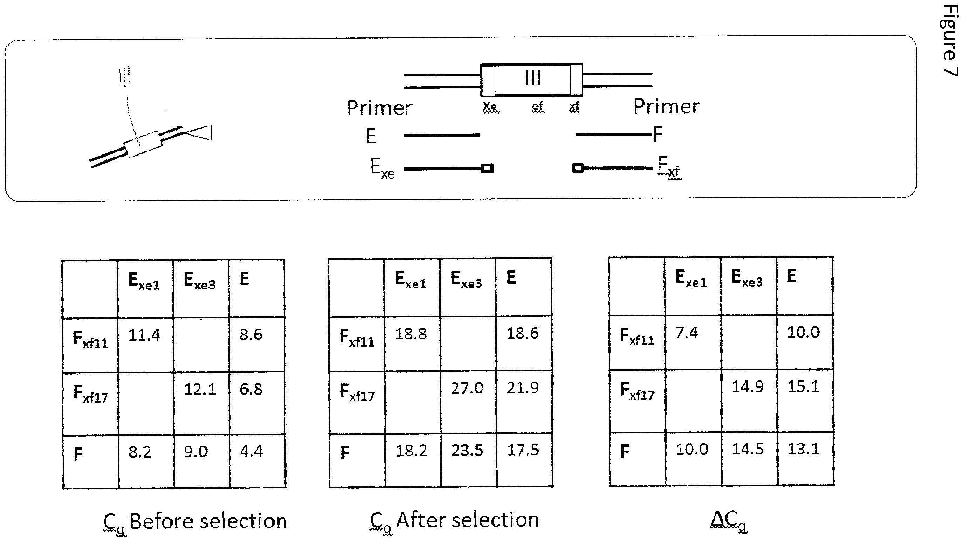

Figure 7 shows a decoding process for a medium DEL having 306 compounds, each tagged with a DNA barcode. Before and after a DEL selection experiment, a primary qPCR was conducted with the primer pairs E and F, with the primer pairs $E_{xe}$ and F, with the primer pairs E and $F_{xf}$, with the primer pairs $E_{xe1}$ and $F_{xf11}$ and with the primer pairs $E_{xe3}$ and $F_{xf17}$. The obtained $C_q$ values before selection are shown in the left matrix, the obtained $C_q$ valued after selection are shown in the middle matrix and the $\Delta C_q$ values are shown in the right matrix in Figure 7. A $\Delta Cq$ value of a primer pair which is below the $\Delta Cq$ value of the primer pair E and F indicates an enrichment of the DNA-substance conjugate. As can be seen in the "$\Delta C_q$"-matrix, subcoding region E-$F_{xf11}$ has $\Delta C_q$-value of 10.0 which is below the $\Delta C_q$-value of 13.1 for subcoding region E-F (i.e. below the control). This means that the subcoding region E-$F_{xf11}$ has been enriched. The same is true for the subcoding region $E_{xe1}$-F with its $\Delta C_q$-value of 10.0 being below the $\Delta C_q$-value of 13.1 for subcoding region E-F (i.e. being below the control). Thus, the results of the primary qPCR indicate that after the DEL selection experiment, substance(s) encoded by the subcoding regions to which primers $E_{xe1}$ and $F_{xf11}$ bind were enriched more strongly than substances encoded by the subcoding regions to which primers $E_{xe3}$ and $F_{xf17}$ bind. Additionally, for confirmation of said data, a secondary qPCR was conducted with the primer pair $E_{xe}$ and $F_{xf}$. Said secondary qPCR confirmed that the subcoding region $E_{xe1}$-$F_{xf11}$ is enriched more strongly than the subcoding region $E_{xe3}$-$F_{xf17}$ (see matrix "$\Delta C_q$" in Figure 7: value in field of column $E_{xe1}$ and row $F_{xf11}$ is much lower than value in field of column $E_{xe3}$ and row $F_{xf17}$ and much lower than the value in the field of column E and row F, which is the control). In summary, both the primary and secondary qPCR demonstrate that the substance(s) connected to the $E_{xe1}$-$F_{xf11}$ sub-coding region must have been strongly enriched after the DEL selection experiment.

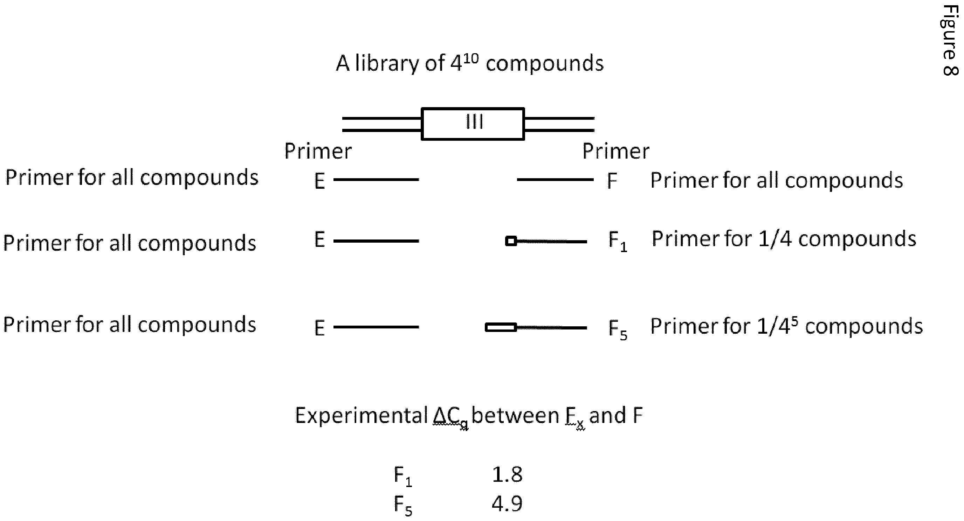

Figure 8 shows an example of a large DEL having $4^{10}$ compounds, each tagged with a DNA barcode. The libraries were generated by partially degenerated synthesis of DNA. Figure 8 illustrates the setup for conducting a primary PCR with one (constant) primer E and various different primers $F_n$, wherein each primer Fn codes for a certain subgroup of the library, specifically $1/4^n$ compounds of the library (having $4^{10}$ compounds in total), wherein n is an integer from 0 to 5. This means that if six primers F are used, the first primer $F_o$ codes for $1/4^0$ of all compounds of the library, i.e. all compounds of the library (= $4^{10}$ = 1048576 compounds), the primer $F_1$ only codes for 1/4 of all compounds of the library (= 262144 compounds), the primer $F_2$ only codes for 1/16 of all compounds of the library (= 65536 compounds), the primer $F_3$ only codes for 1/64 of all compounds of the library (= 16384 compounds), the primer $F_4$ only codes for 1/256 of all compounds of the library (= 4096 compounds) and the primer $F_5$ only codes for 1/1024 of all compounds of the library (= 1024 compounds). This means that after the qPCR has been performed, the group of encoded substances which have been selected in the DEL experiment can be significantly narrowed because encoded substances which are not amplifiable with a primer $F_n$, wherein n is 1 to 5, give no signal in qPCR. For example, if the combination of primer E and primer $F_1$ fails to provide a signal in primary qPCR, it is clear that 3/4 of all compounds, i.e. 786432 compounds of 1048576 compounds, are not amplifiable by said qPCR and thus were not enriched by the DEL selection experiment preceding the primary qPCR. Thus, there are only 1/4 of all compounds (= 262144 compounds) of the DEL library remaining coming into question for having been enriched in the (DEL) selection experiment.

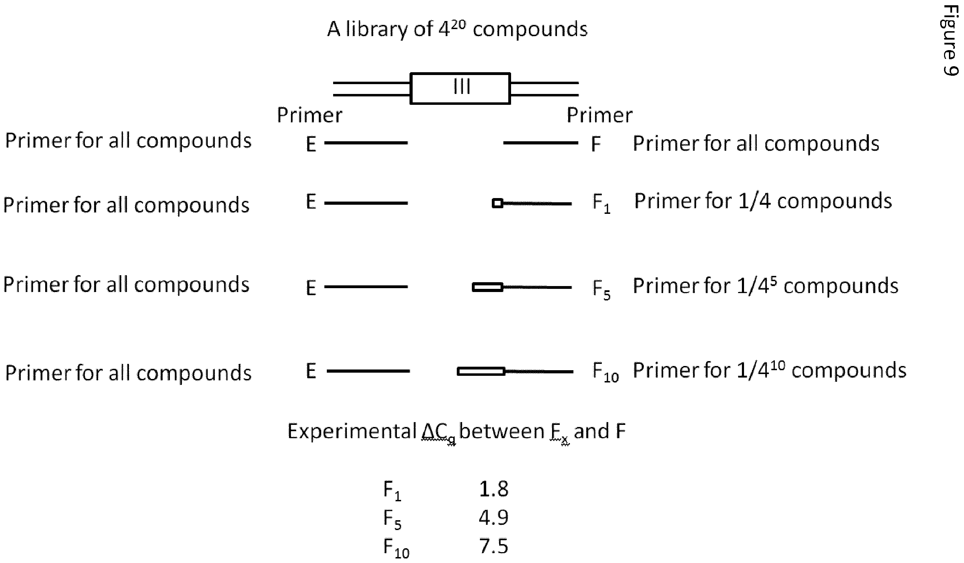

Figure 9 shows another example of a very large DEL having $4^{20}$ compounds, each tagged with a DNA barcode. The principal procedure is the same like the one disclosed in Figure 8 for a DEL having $4^{10}$ compounds. However, due to the larger size of the DEL, it is beneficial if the primary PCR is carried out with more than five different primers F. Specifically, it is beneficial if n is an integer from 0 to 10 in this case. This means that if eleven primers F are used, the

first primer $F_0$ codes for all compounds of the library and the last primer $F_{10}$ only codes for $1/4^{10}$ of all compounds of the library (= 1048576 compounds). This means that after the qPCR has been performed, the group of encoded substances which have been selected in the DEL experiment has been significantly narrowed- For example, a DNA barcode of a DNA-substance conjugate which give no signal with the primer $F_1$ in qPCR means that said DNA barcode belongs to a group of 3/4 of $4^{20}$ compounds ($\approx 8.2 \cdot 10^{11}$ compounds) which have not been enriched in the DEL enrichment experiment. Thus, the group of relevant enriched DNA-substance conjugates has been narrowed to 1/4 of $4^{20}$ compounds ($\approx 2.7 \cdot 10^{11}$ compounds). With each primer $F_n$ increasing from n=1 to n=10, the group of relevant compounds is further narrowed. An amplification signal turning up with primer $F_{10}$ means that the DNA-substance conjugate is within a subgroup of $4^{10}$ ($\approx 1 \cdot 10^6$ compounds) of $4^{20}$ compounds ($\approx 1.1 \cdot 10^{12}$ compounds) in total.

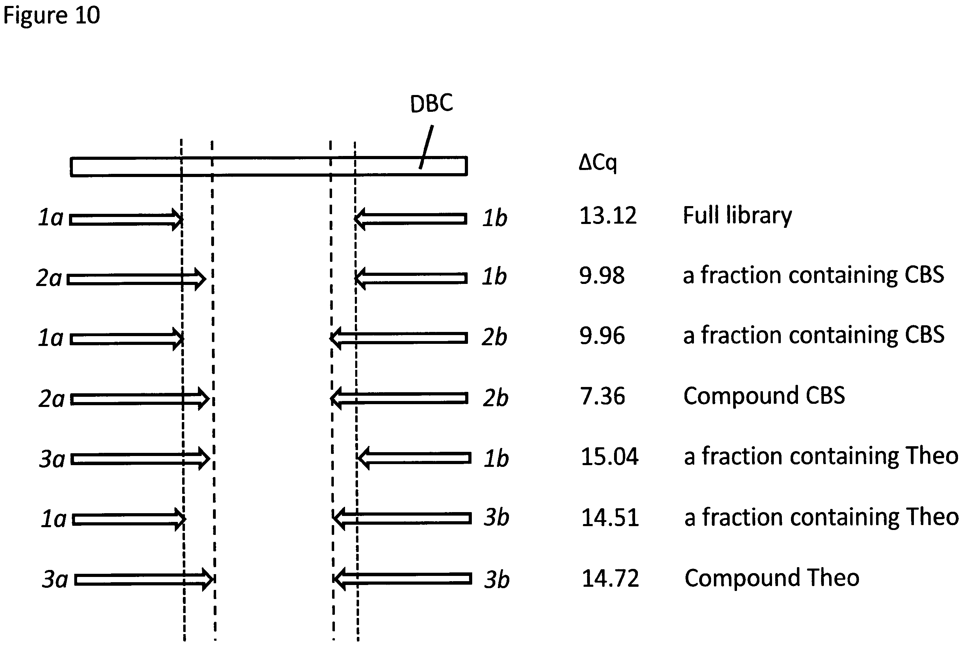

Figure 10 shows the identification of the substance 4-carboxybenzenesulfonamide (in the following: "CBS") after a DNA-CBS-conjugate within a DNA-encoded library (DEL) has been enriched by selection with the enzyme carbonic anhydrase II. A small qPCR matrix has been built using three primers 1a, 2a, 3a pairing with the three primers 1b, 2b, 3b. The primer pair 1a, 1b anneals to all DNA-barcodes of the DNA-substance conjugates of the library and thus has the potential to amplify DNA barcodes of the entire library. Primer 2a covers a sub-library containing DNA-CBS-conjugate and primer 2b covers another sub-library containing DNA-CBS-conjugate. The combination of primers 2a and 2b can be assigned exclusively to CBS. Primer 3a covers a sub-library containing theobromine (in the following: "Theo") conjugated to a DNA barcode (= DNA-Theo-conjugate), primer 3b covers another sub-library containing the DNA-Theo-conjugate. The combination of primers 3a and 3b can be assigned exclusively to Theo. ΔCq is the difference in qPCR cycle before and after selection. A small number reflects large enrichment. ΔCq(1a-1b) > ΔCq(2a-1b) ≈ ΔCq(1a-2b) > ΔCq(2a-2b) indicated that CBS is remarkably enhanced. ΔCq(1a-1b) < ΔCq(3a-1b) ≈ ΔCq(1a-3b) ≈ ΔCq(3a-3b) indicated that Theo is not enriched.

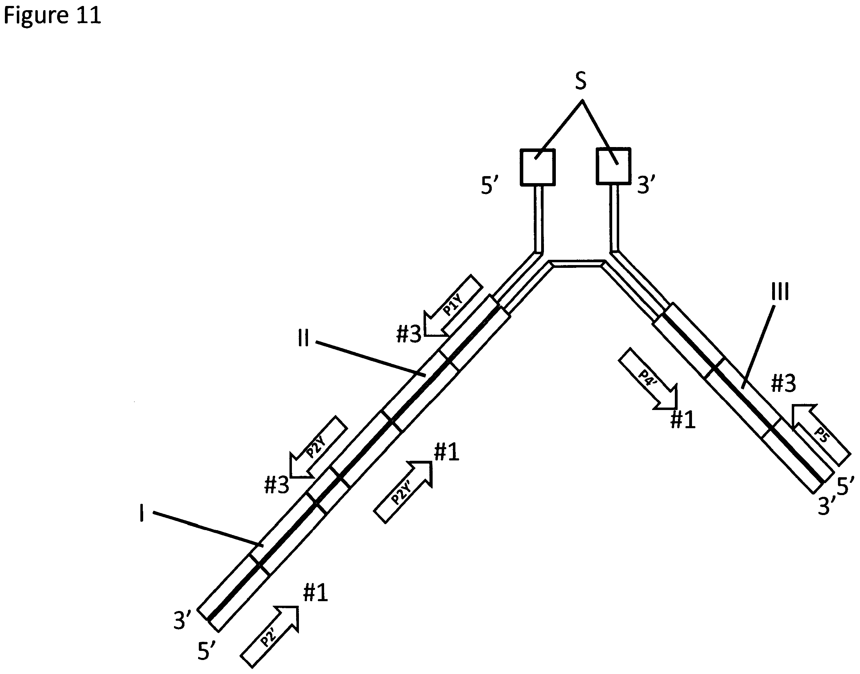

Figure 11 shows a DNA-substance conjugate in which a first substance S is conjugated chemically covalently to a first coding region DNA sequence I and second coding region DNA sequence and in which a second substance S is conjugated chemically covalently to a third coding region DNA sequence III. Each coding region DNA sequence I, II, III has a first part #1 and a third part #3 to which certain primers can bind (i.e. anneal during qPCR). Primer P2' (5'-gctgttccca cattgcgt-3', SEQ-ID Nr. 1) binds to the first part #1 of first coding region DNA sequence I, primer P2Y (5'-ccttctggat tcggtcggag caccatc-3', SEQ-ID Nr. 2) binds to the third part #3 of first coding region DNA sequence I, primer P2Y' (5'-gatggtgctc cgaccgaatc cagaagg-3', SEQ-ID Nr. 3) binds to the first part #1 of second coding region DNA sequence II, primer P1Y (5'-ggaggtgtag acgacagagt atttgactgt cagg-3', SEQ-ID Nr. 4) binds to the third part #3 of second coding region DNA sequence II, primer P4' (5'-cagatcgagc aactccac-3', SEQ-ID Nr. 5) binds to the first part #1 of third coding region DNA sequence III and primer P5 (5'-tggtctcagc cgccctat-3', SEQ-ID Nr. 6) binds to the third part #3 of third coding region DNA sequence III. If substance S has been enriched after a selection experiment with the DNA-encoded library, amplification with primer pair P2' and P2Y, primer pair P2Y' and P1Y and primer pair P4' and P5 each gives a strong amplification signal in qPCR which allows identification of substance S.

**Example 1 - DEL comprising DNA barcodes with one single coding region**

For DNA codes containing only one single coding region, each code has 3 parts, #1 (first part), #2 (second part) and #3 (third part). Each #2 sequence is a unique code, while each combination of #1 and #3 can also represent a unique code (see e.g. Figure 1A). Therefore, a sequence of #2 is corresponding to a combination of #1 and #3.

For each part, there is a minimal difference number n between any pair of sequences (e.g. between two different #1 sequences), while n should be ≥ 2.

**Example 2 - DEL comprising DNA barcodes with two coding regions**

For DNA codes containing two coding regions, each sub-code has 4 parts, for example the first coding region #1 (first part), #2 (second part), #3 (third part) and #4 (fourth part) and the second coding region #1 (first part), #2 (second part), #3 (third part) and #4 (fourth part) (see e.g. Figure 1B). Each #2 (second part) sequence is a unique sub-code, while each combination of #1, #3 and #4 is corresponding to #2 and each combination of #1, #3 and #4 is corresponding to #2.

For each part, there is a minimal difference number n between any pair of sequences (e.g. between two different #1 sequences), while n should be ≥ 2.

**Example 3 - DEL comprising DNA barcodes with more than two coding regions**

For DNA codes containing more than two coding regions (see e.g. Figure 1B or Figure 2), two sub-codes at both ends are designed according Example 2, and the sub-code(s) in between is/are designed according to either Example 1, Example 2 or Example 4. It is very unlikely that a high quality DEL can be synthesized using the split-and-pool method. Therefore, a DEL containing less than 4 sub-codes is favorable.

**Example 4 - DEL comprising DNA barcodes with 5 sub-codes**

The DNA barcodes of this DEL have 5 parts, #1 (first part), #2 (second part), #3 (third part), #4 (fourth part) and #5 (fifth part).

Each #2 sequence (second part) is a unique sub-code, while each combination of #1 and #3 can also represent a unique sub-code. Therefore, a sequence of #2 is corresponding to a combination of #1 and #3. Each combination of #1 and #4 can also represent a unique sub-code. Therefore, a sequence of #2 is corresponding to a combination of #1 and #4. Each combination of #1 and #5 can also represent a unique sub-code. Therefore, a sequence of #2 is corresponding to a combination of #1 and #5.

For each part, there is a minimal difference number n between any pair of sequences (e.g. between two different #1 sequences), while n should be $\geq 2$ in all designs.

**Example 5 - Description of decoding process: Decoding one-(sub)-code**

A primary qPCR matrix is built for the first coding region I using primer A with u different primers B-xb, and primer B with v different primers A-xa. Therefore, the size of resulting matrix is $u \cdot v$ (see e.g. Figure 3). Same matrices can also be built for the second coding region II and the third coding region III.

A secondary qPCR matrix is built for the first coding region I using pairs of B-xb and A-xa, while B-xb and A-xa are chosen according to the signal intensity in the primary matrix. Same secondary matrices can be built for the second coding region II and the third coding region III. The ranking for each building block can thus be concluded.

For sequence containing two sub-codes, an additional secondary qPCR matrix can be built using A-xa and D-xd, while A-xa and D-xd are chosen according to the signal intensity in the primary matrices.

In combination with the two sub-code matrices (A-xa+B-xb and C-xc+D-xd), the ranking of the combinations can be concluded based on certain algorithm, for example:

$$Value^i = Value^i_{matrix\text{-}A+D} \cdot Value^i_{matrix\text{-}A+B} \cdot Value^i_{matrix\text{-}C+D},$$

wherein the Value' is a value relating and being proportional to the amount of a certain DNA barcode in the DEL. In other words, said Value' relates to an individual DNA sequence (barcode structure) which resulted from the combinatorial synthesis through joining two building blocks and two sub-codes.

To further validate the Value' ranking, an additional tertiary qPCR matrix can be built using A-xa-ya and D-xd-yd, while A-xa-ya and D-xd-yd are chosen according to the signal intensity in the primary and secondary matrices and the resulting $Value^i$ ranking.

A full matrix can also be built using A, D and all A-xa-ya and D-xd-yd, though it will be significantly more expensive than the method described before.

The method cannot provide a fully quantitative decoding solution for DEL containing more than two sub-codes. However, combining various primary, secondary, and tertiary rtPCR matrices can provide a $Value^i$ for certain compounds i, which is corresponding to a DNA code containing several sub-codes. All forward and backward primers can be combined to build a matrix.

For example, any primer A, A-xa, A-xa-ya can be combined with any primer B, B-xb, N, N-yn, N-xn-yn, D, D-xd, D-xd-yd to build QPCR matrices. A value for a particular compound can be calculated according to certain algorithm, for example:

$$Value^i = \log_{10}(Value^i_{matrix\text{-}A+D} \cdot Value^i_{matrix\text{-}A+N} \cdot Value^i_{matrix\text{-}M+D} \cdot Value^i_{matrix\text{-}A+B} \cdot Value^i_{matrix\text{-}C+D} \cdot Value^i_{matrix\text{-}M+N})$$

in which the $Value^i_{matrix\text{-}A+D}$, $Value^i_{matrix\text{-}A+N}$ and $Value^i_{matrix\text{-}M+D}$ can be either from the secondary, or tertiary matrices, or as a combination of them, and in which the $Value^i_{matrix\text{-}A+B} \cdot Value^i_{matrix\text{-}C+D} \cdot Value^i_{matrix\text{-}M+N}$ are from the secondary matrices.

List of reference signs

| | |
|---|---|
| DBC: | DNA barcode sequence; |
| S: | substance; |
| I: | first coding region DNA sequence; |
| II: | second coding region DNA sequence; |
| III: | third coding region DNA sequence; |
| #1: | first part of a coding region DNA sequence; |
| #2: | second part of a coding region DNA sequence; |
| #3: | third part of a coding region DNA sequence; |
| #4: | fourth part of a coding region DNA sequence; |
| #5: | fifth part of a coding region DNA sequence; |
| A, B, C, D, E, F, M, N: | primary primer; |
| $A_{xa}$, $B_{xb}$, $C_{xc}$, $D_{xd}$, $E_{xe}$, $F_{xf}$, $M_{xm}$, $N_{yn}$: | secondary primer; |
| $A_{xaya}$, $D_{xdyd}$, $M_{xmym}$, $N_{xnyn}$: | tertiary primer; |
| 1a, 1b: | primary primer binding to all DBS; |
| 2a, 2b: | secondary primer binding to DBS of CBS only; |
| 3a, 3b: | secondary primer binding to Theo only; |
| P2': | primer annealing to first part #1 of coding region I; |
| P2Y: | primer annealing to third part #3 of coding region I; |
| P2Y': | primer annealing to first part #1 of coding region |
| P1Y: | primer annealing to third part #3 of coding region |
| P4': | primer annealing to first part #1 of coding region III; |
| P5: | primer annealing to third part #3 of coding region III. |

**Claims**

1. Method for providing a DNA-encoded library, comprising

    a) synthesizing many different DNA molecules which differ from each other by comprising different DNA barcode sequences, wherein each DNA barcode sequence comprises at least a first coding region DNA sequence comprising at least a first part, a second part and a third part, wherein the second part is located between the first and third part and the second part differs between all the DNA molecules by at least two nucleotides; and
    b) bonding each of the many different DNA molecules to at least a specific substance forming different DNA-substance conjugates, wherein the DNA-substance conjugates differ from each other by the specific substance and by their DNA molecules;

**characterized in that** the first part and the third part encode information regarding the second part of the first coding region, wherein a certain first part and/or a certain third part uniquely codes for a certain group of DNA-substance conjugates which is smaller than the group of all DNA-substance conjugates in the DNA-encoded library.

2. Method according to claim 1, **characterized in that**

   i) the first coding region DNA sequence comprises at least a fourth part, wherein the second part is located between the fourth and third part and wherein both the combination of the first part and the fourth part and the combination of the first part and the third part of the first coding region encode information about the second part of the first coding region; and
   ii) each barcode sequence comprises at least a second coding region DNA sequence comprising at least a first part, a second part, a third part, and a fourth part, wherein the second part is located between the fourth and third part and the second part differs between all the DNA molecules by at least two nucleotides, wherein both the combination of the first part and the fourth part and the combination of the first part and the third part of the second coding region encode information about the second part of the second coding region;

   wherein a certain combination of a first part and fourth part in a certain coding region uniquely codes for a certain group of DNA-substance conjugates which is smaller than the group of all DNA-substance conjugates which is encoded by the first part alone.

3. Method according to one of claims 1 or 2, **characterized in that**

   i) each barcode sequence comprises at least a second coding region DNA sequence comprising at least a first part, a second part, a third part, and a fourth part, wherein the second part is located between the fourth and third part and the second part differs between all the DNA molecules by at least two nucleotides, wherein both the combination of the first part and the fourth part and the combination of the first part and the third part of the second coding region encode information about the second part of the second coding region; and
   ii) each barcode sequence comprises at least a third coding region DNA sequence comprising at least a first part, a second part, a third part, and a fourth part, wherein the second part is located between the fourth and third part and the second part differs between all the DNA molecules by at least two nucleotides, wherein both the combination of the first part and the fourth part and the combination of the first part and third part and the of the third coding region encode information about the second part of the third coding region;

   wherein a certain combination of a first part and fourth part in a certain coding region uniquely codes for a certain group of DNA-substance conjugates which is smaller than the group of DNA-substance conjugates which is encoded by the first part.

4. Method according to one of the preceding claims, **characterized in that**
   at least one coding region DNA sequence, optionally all coding region DNA sequences, comprise at least a first part, a second part, a third part, a fourth part and a fifth part, wherein the second part is located between the fourth and fifth part and the second part differs between all the DNA molecules by at least two nucleotides, wherein the combination of the first part and the fourth part and the combination of the fifth part and the third part of the coding region encode information about the second part of the coding region, preferably of all coding regions, wherein a certain combination of a first part and fourth part uniquely codes for a certain group of DNA-substance conjugates which is smaller than the group of DNA-substance conjugates which is encoded by the first part alone, and wherein a certain combination of a fifth part and third part uniquely codes for a certain group of DNA-substance conjugates which is smaller than the group of DNA-substance conjugates which is encoded by the third part alone.

5. DNA-encoded library, comprising many different DNA-ligand conjugates, wherein the DNA-ligand conjugates differ from each other by their ligand and by their DNA molecules, wherein the DNA molecules of the DNA-ligand conjugates differ from each other by comprising different DNA barcode sequences, wherein each DNA barcode sequence comprises at least a first coding region DNA sequence comprising at least a first part, a second part and a third part, wherein the second part is located between the first and third part and the second part differs between all the DNA molecules by at least two nucleotides; **characterized in that** the first part and the third part encode information regarding the second part of the first coding region, wherein a certain first part and/or a certain third part uniquely codes for a certain group of DNA-ligand conjugates which is smaller than the group of all DNA-ligand conjugates in the DNA-encoded library.

6. DNA-encoded library according to claim 5, **characterized in that**

   i) the first coding region DNA sequence comprises at least a fourth part, wherein the second part is located between the fourth and third part and wherein both the combination of the first part and the fourth part and the combination of the first part and the third part of the first coding region encode information about the second part of the first coding region; and

   ii) each barcode sequence comprises at least a second coding region DNA sequence comprising at least a first part, a second part, a third part, and a fourth part, wherein the second part is located between the fourth and third part and the second part differs between all the DNA molecules by at least two nucleotides, wherein both the combination of the first part and the fourth part and the combination of the first part and the third part of the second coding region encode information about the second part of the second coding region;

   wherein a certain combination of a first part and fourth part in a certain coding region uniquely codes for a certain group of DNA-ligand conjugates which is smaller than the group of all DNA-ligand conjugates which is encoded by the first part alone.

7. DNA-encoded library according to claim 6, **characterized in that** each barcode sequence comprises at least a third coding region DNA sequence, which is on the same DNA strand as the second coding region, comprising at least a first part, a second part, a third part, and a fourth part, wherein the second part is located between the fourth and third part and the second part differs between all the DNA molecules by at least two nucleotides, wherein both the combination of the first part and the fourth part and the combination of the first part and the third part and the of the third coding region encode information about the second part of the third coding region, wherein a certain combination of a first part and fourth part in the second coding region and in the third coding region uniquely codes for a certain group of DNA-ligand conjugates which is smaller than the group of DNA-ligand conjugates which is encoded by the first part alone.

8. DNA-encoded library according to one of claims 5 to 7, **characterized in that** at least one coding region DNA sequence, optionally all coding region DNA sequences, comprise at least a first part, a second part, a third part, a fourth part and a fifth part, wherein the second part is located between the fourth and fifth part and the second part differs between all the DNA molecules by at least two nucleotides,

   wherein the combination of the first part and the fourth part and the combination of the fifth part and the third part of the coding region encode information about the second part of the coding region, preferably of all coding regions, and

   wherein a certain combination of a first part and fourth part uniquely codes for a certain group of DNA-ligand conjugates which is smaller than the group of DNA-ligand conjugates which is encoded by the first part alone and wherein a certain combination of a fifth part and third part uniquely codes for a certain group of DNA-ligand conjugates which is smaller than the group of DNA-ligand conjugates which is encoded by the third part alone.

9. Method of decoding a DNA-encoded library according to one of the claims 5 to 8, comprising

   a) performing a qPCR with the DNA-encoded library according to one of claims 5 to 8 as template, wherein the following primers are used:

   a primer A and a primer B for amplifying the first coding region of every DNA-ligand conjugate; and many different primers A-xN which anneal to the different first parts of the first coding region and many different primers B-yN which anneal to the different third parts of the first coding region, wherein primer A-xN has an identical length like the coding region primer A by shortening x nucleotides at its 5'-end, primer B-yN has an identical length like the coding region primer B by shortening y nucleotides at its 5'-end, N represents a A, T, G or C and x and y represent the total number of any one of A, T, G or C at the 3'-end of the primers, wherein x is an integer from 2 to 6, preferably 4;

   b) calculating a mathematical product of the signal value of each primer A-xN and each primer B-xN by following equation:

   $$\text{Value } (A\text{-}xN)_i = \text{signal value } [(A\text{-}xN)_i + B] \cdot \text{signal value } [(A\text{-}xN)_i + (B\text{-}xN)_i];$$

and

$$\text{Value } (B\text{-}yN)_i = \text{signal value } [(B\text{-}yN)_i + A] \cdot \text{signal value } [(B\text{-}yN)_i + (A\text{-}xn)_i],$$

wherein i is an integer and defines a specific primer, and the "+"-sign indicates a combination of two primers; wherein signal value is the percentage of abundance related to the whole set of qPCR quantification using different primers annealed to the same region; and

c) comparing the obtained mathematical products for each of the primers $(A\text{-}xN)_i$ and $(B\text{-}yN)_i$, wherein those primers with high values code for DNA-ligand conjugates which are present at a high concentration in the DNA-encoded library.

**10.** Method according to claim 9, **characterized in that** the method comprises

i) calculating a mathematical product of the value obtained for each primer A-xN and each primer B-yN by following equation

$$\text{Value } (A\text{-}B)_i = \text{Value } (A\text{-}xN)_i \cdot \text{Value } (B\text{-}yN)_i;$$

ii) comparing the obtained mathematical products for each of the combination of primers $(A\text{-}xN)_i$ and $(B\text{-}yN)_i$, wherein those primer combinations with high values code for DNA-ligand conjugates which are present at a high concentration in the DNA-encoded library.

**11.** Method according to one of claims 9 or 10, **characterized in that** the qPCR is performed with the DNA-encoded library according to one of claims 6 to 8 as template and the method comprises

i) performing a qPCR with the following primers:

a first coding region primer A and a first coding region primer primer B for amplifying the first coding region of every DNA-ligand conjugate; and

many different primers A-xN which anneal to the different first parts, or first and fourth parts of the first coding region and many different primers B-yN which anneal to the different third parts of the first coding region, wherein A-xN has an identical length like the coding region primer A by shortening x nucleotides at its 5'-end, B-yN has an identical length like the coding region primer B by shortening y nucleotides at its 5'-end, N represents a A, T, G or C and x and y represent the total number of any one of A, T, G or C at the 3'-end of the primers, wherein x is an integer from 6 to 10, preferably 8, and y is an integer from 2 to 6, preferably 4; and

a second coding region primer C and a second coding region primer D for amplifying the second coding region of every DNA-ligand conjugate; and

many different primers D-yN which anneal to the different first parts, or first and fourth parts of the second coding region and many different primers C-xN which anneal to the different third parts of the second coding region, wherein primer C-xN has an identical length like the coding region primer C by shortening x nucleotides at its 5'-end, primer D-yN has an identical length like the coding region primer D by shortening y nucleotides at its 5'-end, N represents a A, T, G or C and x and y represent the total number of any one of A, T, G or C at the 3'-end of the primers, wherein x is an integer from 6 to 10, preferably 8, and y is an integer from 2 to 6, preferably 4;

ii) calculating a mathematical product of the signal value of each primer A-xN, each primer B-yN, each primer C-xN and each primer D-yN by following equation:

$$\text{Value } (A\text{-}xN)_i = \text{signal value } [(A\text{-}xN)_i + B] \cdot \text{signal value } [(A\text{-}xN)_i + (B\text{-}xN)_i];$$

$$\text{Value } (B\text{-}yN)_i = \text{signal value } [(B\text{-}yN)_i + A] \cdot \text{signal value } [(B\text{-}yN)_i + (A\text{-}xN)_i],$$

$$\text{Value } (C\text{-}xN)_i = \text{signal value } [(C\text{-}xN)_i + D] \cdot \text{signal value } [(C\text{-}xN)_i + (D\text{-}xn)_i],$$

$$\text{Value } (D\text{-}yN)_i = \text{signal value } [(D\text{-}yN)_i + C] \cdot \text{signal value } [(D\text{-}yN)_i + (C\text{-}xn)_i],$$

wherein i is an integer and defines a specific primer, and the "+"-sign indicates a combination of two primers; wherein signal value is the percentage of abundance related to the whole set of qPCR quantification using different primers annealed to the same region; and

iii) comparing the obtained mathematical products for each of the primers $(A\text{-}xN)_i$, $(B\text{-}yN)_i$, $(C\text{-}xN)_i$ and $(D\text{-}yN)_i$, wherein those primers with high values code for DNA-ligand conjugates which are present at a high concentration in the DNA-encoded library.

**12.** Method according to the preceding claim, **characterized in that** the method comprises

i) calculating a mathematical product of the value obtained for each primer A-xN and each primer B-yN, for each primer A-xN and each primer D-xN and for each primer C-yN and D-xN by following equation

$$\text{Value } (A\text{-}B)_i = \text{Value } (A\text{-}xN)_i \cdot \text{Value } (B\text{-}yN)_i;$$

$$\text{Value } (A\text{-}D)_i = \text{Value } (A\text{-}xN)_i \cdot \text{Value } (D\text{-}yN)_i;$$

$$\text{Value } (C\text{-}D)_i = \text{Value } (C\text{-}xN)_i \cdot \text{Value } (D\text{-}yN)_i;$$

ii) calculating the mathematical product of the Value $(A\text{-}B)_i$, $(A\text{-}D)_i$ and $(C\text{-}D)_i$ for each primer i by the following equation

$$\text{Value}^i = \text{value } (A\text{-}B)_i \cdot \text{value } (A\text{-}D)_i \cdot \text{value } (C\text{-}D)_i$$

iii) comparing the obtained mathematical products $\text{Value}^i$, wherein those primer combinations i with high values code for DNA-ligand conjugates which are present at a high concentration in the DNA-encoded library.

**13.** Method according to one of claims 9 to 12, **characterized in that** the qPCR is performed with the DNA-encoded library according to one of claims 7 or 8 as template and the method comprises

i) performing a qPCR with the following primers:

a first coding region primer A and a first coding region primer B for amplifying the first coding region of every DNA-ligand conjugate; and
many different primers A-xN which anneal to the different first parts, or first and fourth parts of the first coding region, and many different primers B-yN which anneal to the different third parts of the first coding region, wherein A-xN has an identical length like the coding region primer A by shortening x nucleotides at its 5'-end, B has an identical length like the coding region primer B-yN by shortening y nucleotides at its 5'-end, N represents a A, T, G or C and x and y represent the total number of any one of A, T, G or C at the 3'-end of the primers, wherein x is an integer from 6 to 10, preferably 8, and y is an integer from 2 to 6, preferably 4; and
a second coding region primer C and a second coding region primer D for amplifying the second coding region of every DNA-ligand conjugate; and

many different primers D-yN which anneal to the different first parts, or first and fourth parts of the second coding region and many different primers C-xN which anneal to the different third parts of the second coding region, wherein primer C-xN has an identical length like the coding region primer C by shortening x nucleotides at its 5'-end, primer D-yN has an identical length like the coding region primer D by shortening y nucleotides at its 5'-end, N represents a A, T, G or C and x and y represent the total number of any one of A, T, G or C at the 3'-end of the primers, wherein x is an integer from 6 to 10, preferably 8, and y is an integer from 2 to 6, preferably 4;

a third coding region primer E and a third coding region primer F for amplifying the third coding region of every DNA-ligand conjugate; and

many different primers E-xN which anneal to the different first parts of the third coding region and many different primers F-yN which anneal to the different third parts of the third coding region, wherein primer E-xN has an identical length like the coding region primer E by shortening x nucleotides at its 5'-end, primer F-yN has an identical length like the coding region primer F by shortening y nucleotides at its 5'-end, N represents a A, T, G or C and x and y represent the total number of any one of A, T, G or C at the 3'-end of the primers, wherein x is an integer from 6 to 10, preferably 8, and y is an integer from 2 to 6, preferably 4;

ii) calculating a mathematical product of the signal value of each primer A-xN, each primer B-yN, each primer C-xN, each primer D-yN, each primer E-xN and each primer F-yN by following equation:

$$\text{Value } (A\text{-}xN)_i = \text{signal value } [(A\text{-}xN)_i + B] \cdot \text{signal value } [(A\text{-}xN)_i + (B\text{-}xN)_i];$$

$$\text{Value } (B\text{-}yN)_i = \text{signal value } [(B\text{-}yN)_i + A] \cdot \text{signal value } [(B\text{-}yN)_i + (A\text{-}xN)_i],$$

$$\text{Value } (C\text{-}xN)_i = \text{signal value } [(C\text{-}xN)_i + D] \cdot \text{signal value } [(C\text{-}xN)_i + (D\text{-}xN)_i],$$

$$\text{Value } (D\text{-}yN)_i = \text{signal value } [(D\text{-}yN)_i + C] \cdot \text{signal value } [(D\text{-}yN)_i + (C\text{-}xN)_i],$$

$$\text{Value } (E\text{-}xN)_i = \text{signal value } [(E\text{-}xN)_i + F] \cdot \text{signal value } [(E\text{-}xN)_i + (F\text{-}xn)_i],$$

$$\text{Value } (F\text{-}yN)_i = \text{signal value } [(F\text{-}yN)_i + E] \cdot \text{signal value } [(F\text{-}yN)_i + (E\text{-}xN)_i],$$

wherein i is an integer and defines a specific primer, and the "+"-sign indicates a combination of two primers; wherein signal value is the percentage of abundance related to the whole set of qPCR quantification using different primers annealed to the same region; and

iii) comparing the obtained mathematical products for each of the primers $(A\text{-}xN)_i$, $(B\text{-}yN)_i$, $(C\text{-}xN)_i$, $(D\text{-}yN)_i$, $(E\text{-}xN)_i$ and $(N\text{-}yN)_i$, wherein those primers with high values code for DNA-ligand conjugates which are present at a high concentration in the DNA-encoded library.

**14.** Method according to claim 13, **characterized in that** the method comprises

i) calculating a mathematical product of the value obtained for each primer A-xN and each primer B-yN, for each primer A-xN and each primer D-xN, for each primer C-yN and D-xN, for each primer A-xN and N-yN, for each primer M-xN and D-yN and for each primer M-xN and N-yN by following equation

$$\text{Value (A-B)}_i = \text{Value (A-xN)}_i \cdot \text{Value (B-yN)}_i;$$

$$\text{Value (A-D)}_i = \text{Value (A-xN)}_i \cdot \text{Value (D-yN)}_i;$$

$$\text{Value (C-D)}_i = \text{Value (C-xN)}_i \cdot \text{Value (D-yN)}_i;$$

$$\text{Value (A-F)}_i = \text{Value (A-xN)}_i \cdot \text{Value (F-yN)}_i;$$

$$\text{Value (E-D)}_i = \text{Value (E-xN)}_i \cdot \text{Value (D-yN)}_i;$$

$$\text{Value (E-F)}_i = \text{Value (E-xN)}_i \cdot \text{Value (F-yN)}_i;$$

ii) calculating the mathematical product of the values $(A-B)_i$, $(A-D)_i$, $(C-D)_i$, $(A-F)_i$, $(E-D)_i$ and $(E-F)_i$ for each primer combinations i by the following equation

$$\text{Value}^i = \text{value (A-B)}_i \cdot \text{value (A-D)}_i \cdot \text{value (C-D)}_i \cdot \text{value (A-F)}_i \cdot \text{value}$$

$$(E-D)_i \cdot \text{value (E-F)}_i;$$

iii) comparing the obtained mathematical products $\text{Value}^i$, wherein those primer combinations i with high values code for DNA-ligand conjugates which are present at a high concentration in the DNA-encoded library.

**15.** Method according to claim 14, **characterized in that** the method further comprises the calculation of a $\text{Value}^{i'}$ by the following calculation:

$$\text{Value}^{i'} = \log_{10}[\text{value (A-B)}_i \cdot \text{value (A-D)}_i \cdot \text{value (C-D)}_i \cdot \text{value (A-F)}_i \cdot$$

$$\text{value (E-D)}_i \cdot \text{value (E-F)}_i].$$

**Patentansprüche**

**1.** Verfahren zur Bereitstellung einer DNA-kodierten Bibliothek, umfassend

a) Synthetisieren vieler verschiedener DNA-Moleküle, die sich voneinander unterscheiden, indem sie verschiedene DNA-Barcodesequenzen umfassen, wobei jede DNA-Barcodesequenz zumindest eine erste Kodierungsregion-DNA-Sequenz umfasst, die zumindest einen ersten Teil, einen zweiten Teil und einen dritten Teil umfasst, wobei der zweite Teil zwischen dem ersten und dritten Teil angeordnet ist und der zweite Teil sich zwischen allen DNA-Molekülen um zumindest zwei Nukleotide unterscheidet; und
b) Binden jedes der vielen verschiedenen DNA-Moleküle an mindestens eine spezifische Substanz, wodurch verschiedene DNA-Substanz-Konjugate gebildet werden, wobei sich die DNA-Substanz-Konjugate durch die spezifische Substanz und durch ihre DNA-Moleküle voneinander unterscheiden;
**dadurch gekennzeichnet, dass** der erste Teil und der dritte Teil Informationen bezüglich des zweiten Teils der ersten Kodierungsregion kodieren, wobei ein bestimmter erster Teil und/oder ein bestimmter dritter Teil eine bestimmte Gruppe von DNA-Substanz-Konjugaten spezifisch kodiert, die kleiner ist als die Gruppe aller DNA-Substanz-Konjugate in der DNA-kodierten Bibliothek.

**2.** Verfahren nach Anspruch 1, **dadurch gekennzeichnet, dass**

i) die erste Kodierungsregion-DNA-Sequenz zumindest einen vierten Teil umfasst, wobei der zweite Teil zwischen dem vierten und dem dritten Teil angeordnet ist und wobei sowohl die Kombination aus dem ersten Teil und dem vierten Teil als auch die Kombination aus dem ersten Teil und dem dritten Teil der ersten Kodierungsregion Informationen über den zweiten Teil der ersten Kodierungsregion kodieren; und

ii) jede Barcode-Sequenz zumindest eine zweite Kodierungsregion-DNA-Sequenz umfasst, die zumindest einen ersten Teil, einen zweiten Teil, einen dritten Teil und einen vierten Teil umfasst, wobei der zweite Teil zwischen dem vierten und dem dritten Teil angeordnet ist und sich der zweite Teil zwischen all den DNA-Molekülen um zumindest zwei Nukleotide unterscheidet, wobei sowohl die Kombination aus dem ersten Teil und dem vierten Teil als auch die Kombination aus dem ersten Teil und dem dritten Teil der zweiten Kodierungsregion Informationen über den zweiten Teil der zweiten Kodierungsregion kodieren;

wobei eine bestimmte Kombination eines ersten Teils und eines vierten Teils in einer bestimmten Kodierungsregion eine bestimmte Gruppe von DNA-Substanz-Konjugaten spezifisch kodiert, die kleiner ist als die Gruppe aller DNA-Substanz-Konjugate, die durch den ersten Teil allein kodiert werden.

3. Verfahren nach einem der Ansprüche 1 oder 2, **dadurch gekennzeichnet, dass**

i) jede Barcode-Sequenz zumindest eine zweite Kodierungsregion-DNA-Sequenz umfasst, die zumindest einen ersten Teil, einen zweiten Teil, einen dritten Teil und einen vierten Teil umfasst, wobei der zweite Teil zwischen dem vierten und dem dritten Teil angeordnet ist und sich der zweite Teil zwischen all den DNA-Molekülen um zumindest zwei Nukleotide unterscheidet, wobei sowohl die Kombination aus dem ersten Teil und dem vierten Teil als auch die Kombination aus dem ersten Teil und dem dritten Teil der zweiten Kodierungsregion Informationen über den zweiten Teil der zweiten Kodierungsregion kodieren; und

ii) jede Barcode-Sequenz zumindest eine dritte Kodierungsregion-DNA-Sequenz umfasst, die zumindest einen ersten Teil, einen zweiten Teil, einen dritten Teil und einen vierten Teil umfasst, wobei der zweite Teil zwischen dem vierten und dem dritten Teil angeordnet ist und sich der zweite Teil zwischen all den DNA-Molekülen um zumindest zwei Nukleotide unterscheidet, wobei sowohl die Kombination aus dem ersten Teil und dem vierten Teil als auch die Kombination aus dem ersten Teil und dem dritten Teil der dritten Kodierungsregion Informationen über den zweiten Teil der dritten Kodierungsregion kodieren;

wobei eine bestimmte Kombination eines ersten Teils und vierten Teils in einer bestimmten Kodierungsregion eine bestimmte Gruppe von DNA-Substanz-Konjugaten spezifisch kodiert, die kleiner ist als die Gruppe von DNA-Substanz-Konjugaten, die durch den ersten Teil kodiert werden.

4. Verfahren gemäß einem der vorhergehenden Ansprüche, **dadurch gekennzeichnet, dass**
zumindest eine Kodierungsregion-DNA-Sequenz, optional alle Kodierungsregion-DNA-Sequenzen, zumindest einen ersten Teil, einen zweiten Teil, einen dritten Teil, einen vierten Teil und einen fünften Teil umfassen, wobei der zweite Teil zwischen dem vierten und fünften Teil angeordnet ist und der zweite Teil sich zwischen allen DNA-Molekülen um zumindest zwei Nukleotide unterscheidet,
wobei die Kombination aus dem ersten Teil und dem vierten Teil als auch die Kombination aus dem fünften Teil und dem dritten Teil der Kodierungsregion Informationen über den zweiten Teil der Kodierungsregion, vorzugsweise aller Kodierungsregionen, kodieren,
wobei eine bestimmte Kombination eines ersten Teils und vierten Teils eine bestimmte Gruppe von DNA-Substanz-Konjugaten eindeutig kodiert, die kleiner ist als die Gruppe von DNA-Substanz-Konjugaten, die durch den ersten Teil allein kodiert wird, und
wobei eine bestimmte Kombination eines fünften Teils und dritten Teils eine bestimmte Gruppe von DNA-Substanz-Konjugaten eindeutig kodiert, die kleiner ist als die Gruppe von DNA-Substanz-Konjugaten, die durch den dritten Teil allein kodiert wird.

5. DNA-kodierte Bibliothek, umfassend viele verschiedene DNA-Ligand-Konjugate, wobei sich die DNA-Ligand-Konjugate durch ihren Liganden und durch ihre DNA-Moleküle voneinander unterscheiden,
wobei sich die DNA-Moleküle der DNA-Ligand-Konjugate dadurch voneinander unterscheiden, dass sie verschiedene DNA-Barcodesequenzen umfassen, wobei jede DNA-Barcodesequenz zumindest eine erste Kodierungsregion-DNA-Sequenz umfasst, die zumindest einen ersten Teil, einen zweiten Teil und einen dritten Teil umfasst, wobei der zweite Teil zwischen dem ersten und dritten Teil angeordnet ist und der zweite Teil sich zwischen allen DNA-Molekülen um zumindest zwei Nukleotide unterscheidet;
**dadurch gekennzeichnet, dass** der erste Teil und der dritte Teil Informationen bezüglich des zweiten Teils der ersten Kodierungsregion kodieren, wobei ein bestimmter erster Teil und/oder ein bestimmter dritter Teil eine bestimmte Gruppe von DNA-Ligand-Konjugaten eindeutig kodiert, die kleiner ist als die Gruppe aller DNA-Ligand-Konjugate in der DNA-kodierten Bibliothek.

6. DNA-kodierte Bibliothek nach Anspruch 5, **dadurch gekennzeichnet, dass**

i) die erste Kodierungsregion-DNA-Sequenz zumindest einen vierten Teil umfasst, wobei der zweite Teil zwischen dem vierten und dem dritten Teil angeordnet ist und wobei sowohl die Kombination aus dem ersten Teil und dem vierten Teil als auch die Kombination aus dem ersten Teil und dem dritten Teil der ersten Kodierungsregion Informationen über den zweiten Teil der ersten Kodierungsregion kodieren; und
ii) jede Barcode-Sequenz zumindest eine zweite Kodierungsregion-DNA-Sequenz umfasst, die zumindest einen ersten Teil, einen zweiten Teil, einen dritten Teil und einen vierten Teil umfasst, wobei der zweite Teil zwischen dem vierten und dem dritten Teil angeordnet ist und sich der zweite Teil zwischen all den DNA-Molekülen um zumindest zwei Nukleotide unterscheidet, wobei sowohl die Kombination aus dem ersten Teil und dem vierten Teil als auch die Kombination aus dem ersten Teil und dem dritten Teil der zweiten Kodierungsregion Informationen über den zweiten Teil der zweiten Kodierungsregion kodieren;
wobei eine bestimmte Kombination eines ersten Teils und vierten Teils in einer bestimmten Kodierungsregion eine bestimmte Gruppe von DNA-Ligand-Konjugaten eindeutig kodiert, die kleiner ist als die Gruppe aller DNA-Ligand-Konjugate, die durch den ersten Teil allein kodiert werden.

7. DNA-kodierte Bibliothek nach Anspruch 6, **dadurch gekennzeichnet, dass** jede Barcode-Sequenz zumindest eine dritte Kodierungsregion-DNA-Sequenz umfasst, die sich auf demselben DNA-Strang wie die zweite Kodierungsregion befindet, die zumindest einen ersten Teil, einen zweiten Teil, einen dritten Teil und einen vierten Teil umfasst, wobei der zweite Teil zwischen dem vierten und dritten Teil angeordnet ist und sich der zweite Teil zwischen all den DNA-Molekülen um zumindest zwei Nukleotide unterscheidet, wobei sowohl die Kombination aus dem ersten Teil und dem vierten Teil als auch die Kombination aus dem ersten Teil und dem dritten Teil der dritten Kodierungsregion Informationen über den zweiten Teil der dritten Kodierungsregion kodieren, wobei eine bestimmte Kombination eines ersten Teils und vierten Teils in der zweiten Kodierungsregion und in der dritten Kodierungsregion eine bestimmte Gruppe von DNA-Ligand-Konjugaten spezifisch kodiert, die kleiner ist als die Gruppe von DNA-Ligand-Konjugaten, die durch den ersten Teil allein kodiert ist.

8. DNA-kodierte Bibliothek nach einem der Ansprüche 5 bis 7, **dadurch gekennzeichnet, dass** zumindest eine Kodierungsregion-DNA-Sequenz, optional alle Kodierungsregion-DNA-Sequenzen, zumindest einen ersten Teil, einen zweiten Teil, einen dritten Teil, einen vierten Teil und einen fünften Teil umfassen, wobei der zweite Teil zwischen dem vierten und fünften Teil angeordnet ist und der zweite Teil sich zwischen allen DNA-Molekülen um zumindest zwei Nukleotide unterscheidet,
wobei die Kombination aus dem ersten Teil und dem vierten Teil als auch die Kombination aus dem fünften Teil und dem dritten Teil der Kodierungsregion Informationen über den zweiten Teil der Kodierungsregion, vorzugsweise aller Kodierungsregionen, kodieren, und
wobei eine bestimmte Kombination eines ersten Teils und vierten Teils eine bestimmte Gruppe von DNA-Ligand-Konjugaten spezifisch kodiert, die kleiner ist als die Gruppe von DNA-Ligand-Konjugaten, die durch den ersten Teil allein kodiert wird und
wobei eine bestimmte Kombination eines fünften Teils und dritten Teils eine bestimmte Gruppe von DNA-Ligand-Konjugaten spezifisch kodiert, die kleiner ist als die Gruppe von DNA-Ligand-Konjugaten, die durch den dritten Teil allein kodiert wird.

9. Verfahren des Dekodierens einer DNA-kodierten Bibliothek nach einem der Ansprüche 5 bis 8, umfassend

a) Durchführen einer qPCR mit der DNA-kodierten Bibliothek nach einem der Ansprüche 5 bis 8 als Template, wobei die folgenden Primer verwendet werden:

ein Primer A und ein Primer B zum Vervielfältigen der ersten Kodierungsregion jeder DNA-Ligand-Konjugate; und
viele verschiedene Primer A-xN, die an die verschiedenen ersten Teile der ersten Kodierungsregion anlagern, und viele verschiedene Primer B-yN, die an die verschiedenen dritten Teile der ersten Kodierungsregion anlagern,
wobei Primer A-xN eine identische Länge wie der Kodierungsregion-Primer A aufweist, indem x Nukleotide an seinem 5'-Ende gekürzt werden, Primer B-yN eine identische Länge wie der Kodierungsregion-Primer B aufweist, indem y Nukleotide an seinem 5'-Ende gekürzt werden, N ein A, T, G oder C darstellt und x und y die Gesamtzahl von irgendeinem von A, T, G oder C am 3'-Ende der Primer darstellen, wobei x eine ganze Zahl von 2 bis 6, vorzugsweise 4, ist;

b) Berechnen eines mathematischen Produkts des Signalwertes jedes Primers A-xN und jedes Primers B-xN durch folgende Gleichung:

$$\text{Wert } (A\text{-}xN)_i = \text{Signalwert } [(A\text{-}xN)_i + B] \cdot \text{Signalwert } [(A\text{-}xN)_i + (B\text{-}xN)_i];$$

und

$$\text{Wert } (B\text{-}yN)_i = \text{Signalwert } [(B\text{-}yN)_i + A] \cdot \text{Signalwert } [(B\text{-}yN)_i + (A\text{-}xn)_i],$$

wobei i eine ganze Zahl ist und einen spezifischen Primer definiert, und das "+"-Zeichen eine Kombination von zwei Primern anzeigt; wobei Signalwert der Prozentsatz der Häufigkeit ist, bezogen auf den gesamten Satz der qPCR-Quantifizierung unter Verwendung verschiedener Primer, die an dieselbe Region angelagert sind, und

c) Vergleichen der erhaltenen mathematischen Produkte für jeden der Primer $(A\text{-}xN)_i$ und $(B\text{-}yN)_i$, wobei diese Primer mit hohen Werten DNA-Ligand-Konjugate kodieren, die in hoher Konzentration in der DNA-kodierten Bibliothek vorhanden sind.

10. Verfahren nach Anspruch 9, **dadurch gekennzeichnet, dass** das Verfahren umfasst

i) Berechnen eines mathematischen Produkts des Wertes, der für jeden Primer A-xN und jeden Primer B-xN erhalten wird, durch folgende Gleichung

$$\text{Wert } (A\text{-}B)_i = \text{Wert } (A\text{-}xN)_i \cdot \text{Wert } (B\text{-}yN)_i;$$

ii) Vergleichen der erhaltenen mathematischen Produkte für jede der Kombination von Primern $(A\text{-}xN)_i$ und $(B\text{-}yN)_i$, wobei diese Primerkombinationen mit hohen Werten DNA-Ligand-Konjugate kodieren, die in hoher Konzentration in der DNA-kodierten Bibliothek vorhanden sind.

11. Verfahren nach einem der Ansprüche 9 oder 10, **dadurch gekennzeichnet, dass** die qPCR mit der DNA-kodierten Bibliothek nach einem der Ansprüche 6 bis 8 als Template durchgeführt wird und das Verfahren umfasst

i) Durchführen einer qPCR mit den folgenden Primern:

einem ersten Kodierungsregion-Primer A und einem ersten Kodierungsregion-Primer B zum Vervielfältigen der ersten Kodierungsregion jeder DNA-Ligand-Konjugate; und
vielen verschiedenen Primern A-xN, die an die verschiedenen ersten Teile, oder ersten und vierten Teile, der ersten Kodierungsregion anlagern, und vielen verschiedenen Primern B-yN, die an die verschiedenen dritten Teile der ersten Kodierungsregion anlagern, wobei A-xN eine identische Länge wie der Kodierungsregion-Primer A aufweist, indem x Nukleotide an seinem 5'-Ende gekürzt werden, B-yN eine identische Länge wie der Kodierungsregion-Primer B aufweist, indem y Nukleotide an seinem 5'-Ende gekürzt werden, N ein A, T, G oder C darstellt und x und y die Gesamtzahl von irgendeinem von A, T, G oder C am 3'-Ende der Primer darstellen, wobei x eine ganze Zahl von 6 bis 10, vorzugsweise 8, ist und y eine ganze Zahl von 2 bis 6, vorzugsweise 4, ist; und
einem zweiten Kodierungsregion-Primer C und einem zweiten Kodierungsregion-Primer D zum Vervielfältigen der zweiten Kodierungsregion jeder DNA-Ligand-Konjugate; und
vielen verschiedenen Primern D-yN, die an die verschiedenen ersten Teile, oder ersten und vierten Teile, der zweiten Kodierungsregion anlagern, und vielen verschiedenen Primern C-xN, die an die verschiedenen dritten Teile der zweiten Kodierungsregion anlagern, wobei Primer C-xN eine identische Länge wie der Kodierungsregion-Primer C aufweist, indem x Nukleotide an seinem 5'-Ende gekürzt werden, Primer D-yN eine identische Länge wie der Kodierungsregion-Primer D aufweist, indem y Nukleotide an seinem 5'-Ende gekürzt werden, N ein A, T, G oder C darstellt und x und y die Gesamtzahl von irgendeinem von A, T, G oder C am 3'-Ende der Primer darstellen, wobei x eine ganze Zahl von 6 bis 10, vorzugsweise 8, ist und y eine ganze Zahl von 2 bis 6, vorzugsweise 4, ist;

ii) Berechnen eines mathematischen Produkts des Signalwertes jedes Primers A-xN, jedes Primers B-xN, jedes Primers C-xN und jedes Primers D-yN durch folgende Gleichung:

$$\text{Wert } (A\text{-}xN)_i = \text{Signalwert } [(A\text{-}xN)_i+B] \cdot \text{Signalwert } [(A\text{-}xN)_i+(B\text{-}xN)_i];$$

$$\text{Wert } (B\text{-}yN)_i = \text{Signalwert } [(B\text{-}yN)_i+A] \cdot \text{Signalwert } [(B\text{-}yN)_i+(A\text{-}xN)_i],$$

$$\text{Wert } (C\text{-}xN)_i = \text{Signalwert } [(C\text{-}xN)_i+D] \cdot \text{Signalwert } [(C\text{-}xN)_i+(D\text{-}xn)_i],$$

$$\text{Wert } (D\text{-}yN)_i = \text{Signalwert } [(D\text{-}yN)_i+C] \cdot \text{Signalwert } [(D\text{-}yN)_i+(C\text{-}xn)_i],$$

wobei i eine ganze Zahl ist und einen spezifischen Primer definiert, und das "+"-Zeichen eine Kombination von zwei Primern anzeigt; wobei Signalwert der Prozentsatz der Häufigkeit ist, bezogen auf den gesamten Satz der qPCR-Quantifizierung, unter Verwendung verschiedener Primer, die an dieselbe Region angelagert sind, und iii) Vergleichen der erhaltenen mathematischen Produkte für jeden der Primer $(A\text{-}xN)_i$, $(B\text{-}yN)_i$, $(C\text{-}xN)_i$ und $(D\text{-}yN)_i$, wobei diese Primer mit hohen Werten DNA-Ligand-Konjugate kodieren, die in hoher Konzentration in der DNA-kodierten Bibliothek vorhanden sind.

**12.** Verfahren nach dem vorhergehenden Anspruch, **dadurch gekennzeichnet, dass** das Verfahren umfasst

i) Berechnen eines mathematischen Produkts des Wertes, der für jeden Primer A-xN und jeden Primer B-xN, für jeden Primer A-xN und jeden Primer D-xN, und für jeden Primer C-yN und D-xN, erhalten wird, durch folgende Gleichung

$$\text{Wert } (A\text{-}B)_i = \text{Wert } (A\text{-}xN)_i \cdot \text{Wert } (B\text{-}yN)_i;$$

$$\text{Wert } (A\text{-}D)_i = \text{Wert } (A\text{-}xN)_i \cdot \text{Wert } (D\text{-}yN)_i;$$

$$\text{Wert } (C\text{-}D)_i = \text{Wert } (C\text{-}xN)_i \cdot \text{Wert } (D\text{-}yN)_i;$$

ii) Berechnen des mathematischen Produkts des Wertes $(A\text{-}B)_i$, $(A\text{-}D)_i$ and $(C\text{-}D)_i$ für jeden Primer i durch die folgende Gleichung

$$\text{Wert}^i = \text{Wert } (A\text{-}B)_i \cdot \text{Wert } (A\text{-}D)_i \cdot \text{Wert } (C\text{-}D)_i$$

iii) Vergleichen der erhaltenen mathematischen Produkte $\text{Wert}^i$, wobei diese Primerkombinationen i mit hohen Werten DNA-Ligand-Konjugate kodieren, die in hoher Konzentration in der DNA-kodierten Bibliothek vorhanden sind.

**13.** Verfahren nach einem der Ansprüche 9 bis 12, **dadurch gekennzeichnet, dass** die qPCR mit der DNA-kodierten Bibliothek nach einem der Ansprüche 7 oder 8 als Template durchgeführt wird und das Verfahren umfasst

i) Durchführen einer qPCR mit den folgenden Primern:

einem ersten Kodierungsregion-Primer A und einem ersten Kodierungsregion-Primer B zum Vervielfältigen der ersten Kodierungsregion jeder DNA-Ligand-Konjugate; und

vielen verschiedenen Primern A-xN, die an die verschiedenen ersten Teile, oder ersten und vierten Teile, der ersten Kodierungsregion anlagern, und vielen verschiedenen Primern B-yN, die an die verschiedenen dritten Teile der ersten Kodierungsregion anlagern, wobei A-xN eine identische Länge wie der Kodierungsregion-Primer A aufweist, indem x Nukleotide an seinem 5'-Ende gekürzt werden, B eine identische Länge wie der Kodierungsregion-Primer B-yN aufweist, indem y Nukleotide an seinem 5'-Ende gekürzt werden, N ein A, T, G oder C darstellt und x und y die Gesamtzahl von irgendeinem von A, T, G oder C am 3'-Ende der Primer darstellen, wobei x eine ganze Zahl von 6 bis 10, vorzugsweise 8, ist und y eine ganze Zahl von 2 bis 6, vorzugsweise 4, ist; und

einem zweiten Kodierungsregion-Primer C und einem zweiten Kodierungsregion-Primer D zum Vervielfältigen der zweiten Kodierungsregion jeder DNA-Ligand-Konjugate; und

vielen verschiedenen Primern D-yN, die an die verschiedenen ersten Teile, oder ersten und vierten Teile, der zweiten Kodierungsregion anlagern, und vielen verschiedenen Primern C-xN, die an die verschiedenen dritten Teile der zweiten Kodierungsregion anlagern, wobei Primer C-xN eine identische Länge wie der Kodierungsregion-Primer C aufweist, indem x Nukleotide an seinem 5'-Ende gekürzt werden, Primer D-yN eine identische Länge wie der Kodierungsregion-Primer D aufweist, indem y Nukleotide an seinem 5'-Ende gekürzt werden, N ein A, T, G oder C darstellt und x und y die Gesamtzahl von irgendeinem von A, T, G oder C am 3'-Ende der Primer darstellen, wobei x eine ganze Zahl von 6 bis 10, vorzugsweise 8, ist und y eine ganze Zahl von 2 bis 6, vorzugsweise 4, ist;

einem dritten Kodierungsregion-Primer E und einem dritten Kodierungsregion-Primer F zum Vervielfältigen der dritten Kodierungsregion jeder DNA-Ligand-Konjugate; und

vielen verschiedenen Primern E-xN, die an die verschiedenen ersten Teile der dritten Kodierungsregion anlagern und vielen verschiedenen Primern F-yN, die an die verschiedenen dritten Teile der dritten Kodierungsregion anlagern, wobei Primer E-xN eine identische Länge wie der Kodierungsregion-Primer E aufweist, indem x Nukleotide an seinem 5'-Ende gekürzt werden, Primer F-yN eine identische Länge wie der Kodierungsregion-Primer F aufweist, indem y Nukleotide an seinem 5'-Ende gekürzt werden, N ein A, T, G oder C darstellt und x und y die Gesamtzahl von irgendeinem von A, T, G oder C am 3'-Ende der Primer darstellen, wobei x eine ganze Zahl von 6 bis 10, vorzugsweise 8, ist und y eine ganze Zahl von 2 bis 6, vorzugsweise 4, ist;

ii) Berechnen eines mathematischen Produkts des Signalwertes jedes Primers A-xN, jedes Primers B-yN, jedes Primers C-xN, jedes Primers D-yN, jedes Primers E-xN und jedes Primers F-yN durch folgende Gleichung:

$$\text{Wert } (A\text{-}xN)_i = \text{Signalwert } [(A\text{-}xN)_i + B] \cdot \text{Signalwert } [(A\text{-}xN)_i + (B\text{-}xN)_i];$$

$$\text{Wert } (B\text{-}yN)_i = \text{Signalwert } [(B\text{-}yN)_i + A] \cdot \text{Signalwert } [(B\text{-}yN)_i + (A\text{-}xN)_i],$$

$$\text{Wert } (C\text{-}xN)_i = \text{Signalwert } [(C\text{-}xN)_i + D] \cdot \text{Signalwert } [(C\text{-}xN)_i + (D\text{-}xN)_i],$$

$$\text{Wert } (D\text{-}yN)_i = \text{Signalwert } [(D\text{-}yN)_i + C] \cdot \text{Signalwert } [(D\text{-}yN)_i + (C\text{-}xN)_i],$$

$$\text{Wert } (E\text{-}xN)_i = \text{Signalwert } [(E\text{-}xN)_i + F] \cdot \text{Signalwert } [(E\text{-}xN)_i + (F\text{-}xn)_i],$$

$$\text{Wert } (F\text{-}yN)_i = \text{Signalwert } [(F\text{-}yN)_i + E] \cdot \text{Signalwert } [(F\text{-}yN)_i + (E\text{-}xN)_i],$$

wobei i eine ganze Zahl ist und einen spezifischen Primer definiert, und das "+"-Zeichen eine Kombination von zwei Primern anzeigt; wobei Signalwert der Prozentsatz der Häufigkeit ist, bezogen auf den gesamten Satz der qPCR-Quantifizierung, unter Verwendung verschiedener Primer, die an dieselbe Region angelagert sind, und iii) Vergleichen der erhaltenen mathematischen Produkte für jeden der Primer $(A-xN)_i$, $(B-yN)_i$, $(C-xN)_i$, $(D-yN)_i$, $(E-xN)_i$ und $(N-yN)_i$, wobei diese Primer mit hohen Werten DNA-Ligand-Konjugate kodieren, die in hoher Konzentration in der DNA-kodierten Bibliothek vorhanden sind.

**14.** Verfahren nach Anspruch 13, **dadurch gekennzeichnet, dass** das Verfahren umfasst

i) Berechnen eines mathematischen Produkts des für jeden Primer A-xN und jeden Primer B-yN, für jeden Primer A-xN und jeden Primer D-xN, für jeden Primer C-yN und D-xN, für jeden Primer A-xN und N-yN, für jeden Primer M-xN und D-yN und für jeden Primer M-xN und N-yN erhaltenen Wertes durch folgende Gleichung

$$\text{Wert } (A\text{-}B)_i = \text{Wert } (A\text{-}xN)_i \cdot \text{Wert } (B\text{-}yN)_i;$$

$$\text{Wert } (A\text{-}D)_i = \text{Wert } (A\text{-}xN)_i \cdot \text{Wert } (D\text{-}yN)_i;$$

$$\text{Wert } (C\text{-}D)_i = \text{Wert } (C\text{-}xN)_i \cdot \text{Wert } (D\text{-}yN)_i;$$

$$\text{Wert } (A\text{-}F)_i = \text{Wert } (A\text{-}xN)_i \cdot \text{Wert } (F\text{-}yN)_i;$$

$$\text{Wert } (E\text{-}D)_i = \text{Wert } (E\text{-}xN)_i \cdot \text{Wert } (D\text{-}yN)_i;$$

$$\text{Wert } (E\text{-}F)_i = \text{Wert } (E\text{-}xN)_i \cdot \text{Wert } (F\text{-}yN)_i;$$

ii) Berechnen des mathematischen Produkts der Werte $(A\text{-}B)i$, $(A\text{-}D)_i$, $(C\text{-}D)_i$, $(A\text{-}F)_i$, $(E\text{-}D)_i$ und $(E\text{-}F)_i$, für jede Primerkombination i durch die folgende Gleichung

$$\text{Wert}^i = \text{Wert } (A\text{-}B)_i \cdot \text{Wert } (A\text{-}D)_i \cdot \text{Wert } (C\text{-}D)_i \cdot \text{Wert } (A\text{-}F)_i \cdot \text{Wert } (E\text{-}D)_i \cdot \text{Wert } (E\text{-}F)_i;$$

iii) Vergleichen der erhaltenen mathematischen Produkte $\text{Wert}^i$, wobei diese Primerkombinationen i mit hohen Werten DNA-Ligand-Konjugate kodieren, die in hoher Konzentration in der DNA-kodierten Bibliothek vorhanden sind.

**15.** Verfahren nach Anspruch 14, **dadurch gekennzeichnet, dass** das Verfahren ferner die Berechnung eines $\text{Wertes}^{i'}$ durch die folgende Berechnung umfasst:

$$\text{Wert}^{i'} = \log_{10}[\text{Wert } (A\text{-}B)_i \cdot \text{Wert } (A\text{-}D)_i \cdot \text{Wert } (C\text{-}D)_i \cdot \text{Wert } (A\text{-}F)_i \cdot \text{Wert } (E\text{-}D)_i \cdot \text{Wert } (E\text{-}F)_i].$$

**Revendications**

**1.** Procédé de fourniture d'une bibliothèque codée par ADN, comprenant les étapes consistant à :

a) synthétiser de nombreuses molécules d'ADN différentes qui diffèrent les unes des autres en ce qu'elles comprennent différentes séquences de codes barres d'ADN, dans lequel chaque séquence de code barre d'ADN comprend au moins une séquence d'ADN de la première région de codage, comprenant au moins une première partie, une deuxième partie et une troisième partie, dans lequel la deuxième partie est située entre la première et la troisième partie et la deuxième partie diffère de toutes les molécules d'ADN d'au moins deux nucléotides ; et

b) lier chacune des nombreuses molécules d'ADN différentes à au moins une substance spécifique en formant différents conjugués substance-ADN, dans lequel les conjugués substance-ADN diffèrent les uns des autres par la substance spécifique et par leurs molécules d'ADN ;

**caractérisé en ce que** la première partie et la troisième partie codent les informations relatives à la deuxième partie de la première région de codage, dans lequel une certaine première partie et/ou une certaine troisième partie codent uniquement pour un certain groupe de conjugués substance-ADN qui est inférieur au groupe de tous les conjugués substance-ADN dans la bibliothèque codée par ADN.

2. Procédé selon la revendication 1, **caractérisé en ce que**

i) la séquence d'ADN de la première région de codage comprend au moins une quatrième partie, dans laquelle la deuxième partie est située entre la quatrième et la troisième parties et dans lequel la combinaison de la première partie et de la quatrième partie et la combinaison de la première partie et de la troisième partie de la première région de codage codent les informations relatives à la deuxième partie de la première région de codage ; et

ii) chaque séquence de code barre comprend au moins une séquence d'ADN d'une deuxième région de codage comprenant au moins une première partie, une deuxième partie, une troisième partie et une quatrième partie, dans laquelle la deuxième partie est située entre la quatrième et la troisième parties et la deuxième partie diffère de toutes les molécules d'ADN d'au moins deux nucléotides, dans lequel la combinaison de la première partie et de la quatrième partie et la combinaison de la première partie et de la troisième partie de la deuxième région de codage codent des informations relatives à la deuxième partie de la deuxième région de codage ;

dans lequel une certaine combinaison d'une première partie et d'une quatrième partie dans une certaine région de codage code uniquement pour un certain groupe de conjugués substance-ADN qui est plus petit que le groupe de tous les conjugués substance-ADN qui est codé par la première partie seule.

3. Procédé selon l'une quelconque des revendications 1 ou 2, **caractérisé en ce que**

i) chaque séquence de code barre comprend au moins une séquence d'ADN d'une deuxième région de codage comprenant au moins une première partie, une deuxième partie, une troisième partie et une quatrième partie, dans lequel la deuxième partie est située entre la quatrième et la troisième parties et la deuxième partie diffère de toutes les autres molécules d'ADN d'au moins deux nucléotides, dans lequel la combinaison de la première partie et de la quatrième partie et la combinaison de la première partie et de la troisième partie de la deuxième région de codage codent des informations relatives à la deuxième partie de la deuxième région de codage ; et

ii) chaque séquence de code barre comprend au moins une séquence d'ADN d'une troisième région de codage comprenant au moins une première partie, une deuxième partie, une troisième partie, et une quatrième partie, dans lequel la deuxième partie est située entre la quatrième et la troisième partie et la deuxième partie diffère de toutes les molécules d'ADN d'au moins deux nucléotides, dans lequel la combinaison de la première partie et de la quatrième partie et la combinaison de la première partie et de la troisième partie de la troisième région de codage codent des informations relatives à la deuxième partie de la troisième région de codage ;

dans lequel une certaine combinaison d'une première partie et d'une quatrième partie dans une certaine région de codage code uniquement pour un certain groupe de conjugués substance-ADN qui est plus petit que le groupe de conjugués substance-ADN qui est codé par la première partie.

4. Procédé selon l'une des revendications précédentes, **caractérisé en ce que**
au moins une séquence d'ADN d'une région de codage, éventuellement les séquences d'ADN de toutes les régions de codage comprennent au moins une première partie, une deuxième partie, une troisième partie, une quatrième partie et une cinquième partie, dans lequel la deuxième partie est située entre la quatrième et la cinquième parties et la deuxième partie diffère de toutes les molécules d'ADN d'au moins deux nucléotides,

dans lequel la combinaison de la première partie et de la quatrième partie et la combinaison de la cinquième partie et de la troisième partie de la région de codage codent des informations relatives à la deuxième partie de la région de codage, de préférence de toutes les régions de codage,

dans lequel une certaine combinaison d'une première partie et d'une quatrième partie code uniquement pour un certain groupe de conjugués substance-ADN qui est plus petit que le groupe de conjugués substance-ADN qui est codé par la première partie seule, et

dans lequel une certaine combinaison d'une cinquième partie et d'une troisième partie code uniquement pour un certain groupe de conjugués substance-ADN qui est plus petit que le groupe de conjugués substance-ADN qui est codé par la troisième partie seule.

**5.** Bibliothèque codée par ADN, comprenant de nombreux conjugués ligand-ADN différents, dans laquelle les conjugués ligand-ADN diffèrent les uns des autres par leur ligand et par leurs molécules d'ADN,
dans laquelle les molécules d'ADN des conjugués ligand-ADN diffèrent les unes des autres en ce qu'elles comprennent différentes séquences de code barre d'ADN, dans laquelle chaque séquence de code barre d'ADN comprend au moins une séquence d'ADN d'une première région de codage comprenant au moins une première partie, une deuxième partie et une troisième partie, dans laquelle la deuxième partie est située entre la première et la troisième parties et la deuxième partie diffère de toutes les molécules d'ADN d'au moins deux nucléotides ;
**caractérisée en ce que** la première partie et la troisième partie codent des informations relatives à la deuxième partie de la première région de codage, dans laquelle une certaine première partie et/ou une certaine troisième partie code.nt uniquement pour un certain groupe de conjugués ligand-ADN qui est plus petit que le groupe de tous les conjugués ligand-ADN dans la bibliothèque codée par ADN.

**6.** Bibliothèque codée par ADN selon la revendication 5, **caractérisée en ce que** :

i) la séquence d'ADN de la première région de codage comprend au moins une quatrième partie, dans laquelle la deuxième partie est située entre la quatrième et la troisième parties et dans lequel la combinaison de la première partie et de la quatrième partie et la combinaison de la première partie et de la troisième partie de la première région de codage codent les informations relatives à la deuxième partie de la première région de codage ; et

ii) chaque séquence de code barre comprend au moins une séquence d'ADN d'une deuxième région de codage comprenant au moins une première partie, une deuxième partie, une troisième partie et une quatrième partie, dans laquelle la deuxième partie est située entre la quatrième et la troisième parties et la deuxième partie diffère de toutes les molécules d'ADN d'au moins deux nucléotides, dans lequel la combinaison de la première partie et de la quatrième partie et la combinaison de la première partie et de la troisième partie de la deuxième région de codage codent des informations relatives à la deuxième partie de la deuxième région de codage ;
dans laquelle une certaine combinaison d'une première partie et d'une quatrième partie dans une certaine région de codage code uniquement pour un certain groupe de conjugués ligand-ADN qui est plus petit que le groupe de tous les conjugués ligand-ADN qui est codé par la première partie seule.

**7.** Bibliothèque codée par ADN selon la revendication 6, **caractérisée en ce que** chaque séquence de code barre comprend au moins une séquence d'ADN d'une troisième région de codage, qui est sur le même brin d'ADN que la deuxième région de codage, comprenant au moins une première partie, une deuxième partie, une troisième partie et une quatrième partie, dans laquelle la deuxième partie est située entre la quatrième et la troisième parties et la deuxième partie diffère de toutes les molécules d'ADN d'au moins deux nucléotides, dans laquelle la combinaison de la première partie et de la quatrième partie et la combinaison de la première partie et de la troisième partie de la troisième région de codage codent des informations relatives à la deuxième partie de la troisième région de codage, dans laquelle une certaine combinaison d'une première partie et d'une quatrième partie dans la deuxième région de codage et dans la troisième région de codage code uniquement pour un certain groupe de conjugués ligand-ADN qui est plus petit que le groupe de conjugués ligand-ADN qui est codé par la première partie seule.

**8.** Bibliothèque codée par ADN selon l'une des revendications 5 à 7, **caractérisée en ce qu'**au moins une séquence d'ADN d'une région de codage, éventuellement les séquences d'ADN de toutes les régions de codage, comprennent au moins une première partie, une deuxième partie, une troisième partie, une quatrième partie et une cinquième partie, dans laquelle la deuxième partie est située entre la quatrième et la cinquième parties et la deuxième partie diffère de toutes les molécules d'ADN d'au moins deux nucléotides,
dans laquelle la combinaison de la première partie et de la quatrième partie et la combinaison de la cinquième partie et de la troisième partie de la région de codage codent des informations relatives à la deuxième partie de la région de codage, de préférence de toutes les régions de codage, et
dans laquelle une certaine combinaison d'une première partie et d'une quatrième partie code uniquement pour un certain groupe de conjugués ligand-ADN qui est plus petit que le groupe de conjugués ligand-ADN qui est codé par la première partie seule et
dans laquelle une certaine combinaison d'une cinquième partie et d'une troisième partie code uniquement pour un certain groupe de conjugués ligand-ADN qui est plus petit que le groupe de conjugués ligand-ADN qui est codé par la troisième partie seule.

**9.** Procédé de décodage d'une bibliothèque codée par ADN selon l'une des revendications 5 à 8, comprenant les étapes consistant à :

a) réaliser un qPCR avec la bibliothèque codée par ADN selon l'une des revendications 5 à 8 comme modèle, dans lequel les amorces suivantes sont utilisées :

une amorce A et une amorce B pour amplifier la première région de codage de chaque conjugué ligand-ADN ; et
de nombreuses amorces différentes A-xN qui se fixent par annelage aux différentes premières parties de la première région de codage et de nombreuses amorces différentes B-yN qui se fixent par annelage aux différentes troisièmes parties de la première région de codage,
dans lequel une amorce A-xN présente une longueur identique à l'amorce de la région de codage A en raccourcissant x nucléotides à son extrémité 5', une amorce B-yN présente une longueur identique à l'amorce B de la région de codage en raccourcissant y nucléotides à son extrémité 5', N représente A, T, G ou C et x et y représentent le nombre total de A, T, G ou C à l'extrémité 3' des amorces, dans lequel x est un nombre entier de 2 à 6, de préférence égal à 4 ;

b) calculer un produit mathématique de la valeur de signal de chaque amorce A-xN et de chaque amorce B-xN par l'équation suivante :

$$\text{Valeur } (A\text{-}xN)_i = \text{valeur de signal } [(A\text{-}xN)_i + B] \cdot \text{valeur de signal}$$

$$[(A\text{-}xN)_i + (B\text{-}xN)_i] ;$$

et

$$\text{Valeur } (B\text{-}yN)_i = \text{valeur de signal } [(B\text{-}yN)_i + A] \cdot \text{valeur de signal}$$

$$[(B\text{-}yN)_i + (A\text{-}xn)_i] ;$$

dans lequel i est un nombre entier et définit une amorce spécifique, et le signe « + » indique une combinaison de deux amorces ; dans lequel la valeur de signal est le pourcentage d'abondance lié à l'ensemble de quantification qPCR en utilisant différentes amorces fixées par annelage à la même région ; et
c) comparer les produits mathématiques obtenus pour chacune des amorces (A-xN)_i et (B-yN)_i, dans lequel les amorces avec des valeurs élevées codent pour des conjugués ligand-ADN qui sont présents en concentration élevée dans la bibliothèque codée par ADN.

**10.** Procédé selon la revendication 9, **caractérisé en ce que** le procédé comprend les étapes consistant à :

i) calculer un produit mathématique de la valeur obtenue pour chaque amorce A-xN et chaque amorce B-yN par l'équation suivante :

$$\text{Valeur } (A\text{-}B)_i = \text{valeur } (A\text{-}xN)_i \cdot \text{valeur } (B\text{-}yN)_i ;$$

ii) comparer les produits mathématiques obtenus pour chacune des combinaisons d'amorces (A-xN)_i et (B-yN)_i, dans lequel les combinaisons d'amorces avec des valeurs élevées codent pour des conjugués ligand-ADN qui sont présents en concentration élevée dans la bibliothèque codée par ADN.

**11.** Procédé selon l'une des revendications 9 ou 10, **caractérisé en ce que** le qPCR est réalisé avec la bibliothèque codée par ADN selon l'une des revendications 6 à 8 comme modèle et le procédé comprend les étapes consistant à :

i) réaliser un qPCR avec les amorces suivantes :

une amorce A d'une première région de codage et une amorce B d'une première région de codage pour amplifier la première région de codage de chaque conjugué ligand-ADN ; et
de nombreuses amorces différentes A-xN qui se fixent par annelage aux différentes premières parties, ou aux première et quatrième parties de la première région de codage et de nombreuses amorces différentes B-yN qui se fixent par annelage aux différentes troisièmes parties de la première région de codage, dans lequel A-xN présente une longueur identique à l'amorce A de la région de codage en raccourcissant x nucléotides à son extrémité 5', B-yN présente une longueur identique à l'amorce B de la région de codage en raccourcissant y nucléotides à son extrémité 5', N représente A, T, G ou C et x et y représentent le nombre total de A, T, G ou C à l'extrémité 3' des amorces, dans lequel x est un nombre entier de 6 à 10, de préférence égal à 8, et y est un nombre entier de 2 à 6, de préférence égal à 4 ; et

une amorce C d'une deuxième région de codage et une amorce D d'une deuxième région de codage pour amplifier la deuxième région de codage de chaque conjugué ligand-ADN ; et

de nombreuses amorces différentes D-yN qui se fixent par annelage aux différentes premières parties, ou aux première et quatrième parties de la deuxième région de codage et de nombreuses amorces différentes C-xN qui se fixent par annelage aux différentes troisièmes parties de la deuxième région de codage, dans lequel l'amorce C-xN présente une longueur identique à l'amorce C de la région de codage en raccourcissant x nucléotides à son extrémité 5', l'amorce D-yN présente une longueur identique à l'amorce D de la région de codage en raccourcissant y nucléotides à son extrémité 5', N représente A, T, G ou C et x et y représentent le nombre total de A, T, G ou C à l'extrémité 3' des amorces, dans lequel x est un nombre entier de 6 à 10, de préférence égal à 8, et y est un nombre entier de 2 à 6, de préférence égal à 4 ;

ii) calculer un produit mathématique de la valeur de signal de chaque amorce A-xN, de chaque amorce B-yN, de chaque amorce C-xN et de chaque amorce D-yN, par l'équation suivante :

$$\text{Valeur } (A\text{-}xN)_i = \text{valeur de signal } [(A\text{-}xN)_i + B] \cdot \text{valeur de signal } [(A\text{-}xN)_i + (B\text{-}xN)_i] ;$$

$$\text{Valeur } (B\text{-}yN)_i = \text{valeur de signal } [(B\text{-}yN)_i + A] \cdot \text{valeur de signal } [(B\text{-}yN)_i + (A\text{-}xN)_i] ;$$

$$\text{Valeur } (C\text{-}xN)_i = \text{valeur de signal } [(C\text{-}xN)_i + D] \cdot \text{valeur de signal } [(C\text{-}xN)_i + (D\text{-}xn)_i] ;$$

$$\text{Valeur } (D\text{-}yN)_i = \text{valeur de signal } [(D\text{-}yN)_i + C] \cdot \text{valeur de signal } [(D\text{-}yN)_i + (C\text{-}xn)_i] ;$$

dans lequel i est un nombre entier et définit une amorce spécifique, et le signe « + » indique une combinaison de deux amorces ; dans lequel la valeur de signal est le pourcentage d'abondance lié à l'ensemble de la quantification qPCR en utilisant différentes amorces fixées par annelage à la même région ; et

iii) comparer les produits mathématiques obtenus pour chacune des amorces $(A\text{-}xN)_i$, $(B\text{-}yN)_i$, $(C\text{-}xN)_i$ et $(D\text{-}yN)_i$, dans lequel les amorces avec des valeurs élevées codent pour les conjugués ligand-ADN qui sont présents en concentration élevée dans la bibliothèque codée par ADN.

12. Procédé selon la revendication précédente, **caractérisé en ce que** le procédé comprend les étapes consistant à :

i) calculer un produit mathématique de la valeur obtenue pour chaque amorce A-xN et chaque amorce B-yN, pour chaque amorce A-xN et chaque amorce D-xN et pour chaque amorce C-yN et D-xN, par l'équation suivante :

$$\text{Valeur } (A\text{-}B)_i = \text{valeur } (A\text{-}xN)_i \cdot \text{valeur } (B\text{-}yN)_i ;$$

$$\text{Valeur } (A\text{-}D)_i = \text{valeur } (A\text{-}xN)_i \cdot \text{valeur } (D\text{-}yN)_i ;$$

$$\text{Valeur } (C\text{-}D)_i = \text{valeur } (C\text{-}xN)_i \cdot \text{valeur } (D\text{-}yN)_i ;$$

ii) calculer le produit mathématique de la valeur $(A\text{-}B)_i$, $(A\text{-}D)_i$ et $(C\text{-}D)_i$ pour chaque amorce par l'équation suivante :

$$\text{Valeur}^i = \text{valeur } (A\text{-}B)_i \cdot \text{valeur } (A\text{-}D)_i \cdot \text{valeur } (C\text{-}D)_i$$

iii) comparer les produits mathématiques obtenus Valeur$^i$, dans lequel les combinaisons d'amorces i avec des valeurs élevées codent pour des conjugués ligand-ADN qui sont présents en concentration élevée dans la bibliothèque codée par ADN.

**13.** Procédé selon l'une quelconque des revendications 9 à 12, **caractérisé en ce que** le qPCR est réalisé avec la bibliothèque codée par ADN selon l'une des revendications 7 ou 8 comme modèle et le procédé comprend les étapes consistant à

i) réaliser un qPCR avec les amorces suivantes :

une amorce A d'une première région de codage et une amorce B d'une première région de codage pour amplifier la première région de codage de chaque conjugué ligand-ADN ; et
de nombreuses amorces différentes A-xN qui se fixent par annelage aux différentes premières parties, ou aux première et quatrième parties de la première région de codage et de nombreuses amorces différentes B-yN qui se fixent par annelage aux différentes troisièmes parties de la première région de codage, dans lequel A-xN présente une longueur identique à l'amorce A de la région de codage en raccourcissant x nucléotides à son extrémité 5', B présente une longueur identique à l'amorce B-yN de la région de codage en raccourcissant y nucléotides à son extrémité 5', N représente A, T, G ou C et x et y représentent le nombre total de A, T, G ou C à l'extrémité 3' des amorces, dans lequel x est un nombre entier de 6 à 10, de préférence égal à 8, et y est un nombre entier de 2 à 6, de préférence égal à 4 ; et
une amorce C d'une deuxième région de codage et une amorce D d'une deuxième région de codage pour amplifier la deuxième région de codage de chaque conjugué ligand-ADN ; et
de nombreuses amorces différentes D-yN qui se fixent par annelage aux différentes premières parties, ou aux première et quatrième parties de la deuxième région de codage et de nombreuses amorces différentes C-xN qui se fixent par annelage aux différentes troisièmes parties de la deuxième région de codage, dans lequel l'amorce C-xN présente une longueur identique à l'amorce C de la région de codage en raccourcissant x nucléotides à son extrémité 5', l'amorce D-yN présente une longueur identique à l'amorce D de la région de codage en raccourcissant y nucléotides à son extrémité 5', N représente A, T, G ou C et x et y représentent le nombre total de A, T, G ou C à l'extrémité 3' des amorces, dans lequel x est un nombre entier de 6 à 10, de préférence égal à 8, et y est un nombre entier de 2 à 6, de préférence égal à 4 ;
une amorce E d'une troisième région de codage et une amorce F d'une troisième région de codage pour amplifier la troisième région de codage de chaque conjugué ligand-ADN ; et
de nombreuses amorces différentes E-xN qui se fixent par annelage aux différentes premières parties de la troisième région de codage et de nombreuses amorces différentes F-yN qui se fixent par annelage aux différentes troisièmes parties de la troisième région de codage, dans lequel l'amorce E-xN présente une longueur identique à l'amorce E de la région de codage en raccourcissant x nucléotides à son extrémité 5', l'amorce F-yN présente une longueur identique à l'amorce F de la région de codage en raccourcissant y nucléotides à son extrémité 5', N représente A, T, G ou C et x et y représentent le nombre total de A, T, G ou C à l'extrémité 3' des amorces, dans lequel x est un nombre entier de 6 à 10, de préférence égal à 8, et y est un nombre entier de 2 à 6, de préférence égal à 4 ;

ii) calculer un produit mathématique de la valeur de signal de chaque amorce A-xN, de chaque amorce B-yN, de chaque amorce C-xN, de chaque amorce D-yN, de chaque amorce E-xN et de chaque amorce F-yN, par l'équation suivante :

$$\text{Valeur } (A\text{-}xN) = \text{valeur de signal } [(A\text{-}xN)_i + B] \cdot \text{valeur de signal}$$
$$[(A\text{-}xN)_i + (B\text{-}xN)_i] \, ;$$

$$\text{Valeur } (B\text{-}yN)_i = \text{valeur de signal } [(B\text{-}yN)_i + A] \cdot \text{valeur de signal}$$
$$[(B\text{-}yN)_i + (A\text{-}xN)_i] \, ;$$

$$\text{Valeur (C-xN)}_i = \text{valeur de signal } [(C\text{-}xN)_i + D] \cdot \text{valeur de signal}$$
$$[(C\text{-}xN)_i + (D\text{-}xN)_i] \ ;$$

$$\text{Valeur (D-yN)}_i = \text{valeur de signal } [(D\text{-}yN)_i + C] \cdot \text{valeur de signal}$$
$$[(D\text{-}yN)_i + (C\text{-}xN)_i] \ ;$$

$$\text{Valeur (E-xN)}_i = \text{valeur de signal } [(E\text{-}xN)_i + F] \cdot \text{valeur de signal}$$
$$[(E\text{-}xN)_i + (F\text{-}xn)_i] \ ;$$

$$\text{Valeur (F-yN)}_i = \text{valeur de signal } [(F\text{-}yN)_i + E] \cdot \text{valeur de signal}$$
$$[(F\text{-}yN)_i + (E\text{-}xN)_i] \ ;$$

dans lequel i est un nombre entier et définit une amorce spécifique, et le signe « + » indique une combinaison de deux amorces ; dans lequel la valeur de signal est le pourcentage d'abondance lié à l'ensemble de la quantification qPCR en utilisant différentes amorces fixées par annelage à la même région ; et

iii) comparer les produits mathématiques obtenus pour chacune des amorces $(A\text{-}xN)_i$, $(B\text{-}yN)_i$, $(C\text{-}xN)_i$, $(D\text{-}yN)_i$, $(E\text{-}xN)_i$, et $(N\text{-}yN)_i$, dans lequel les amorces avec des valeurs élevées codent pour les conjugués ligand-ADN qui sont présents en concentration élevée dans la bibliothèque codée par ADN.

14. Procédé selon la revendication 13, **caractérisé en ce que** le procédé comprend les étapes consistant à :

i) calculer un produit mathématique de la valeur obtenue pour chaque amorce A-xN et chaque amorce B-yN, pour chaque amorce A-xN et chaque amorce D-yN, pour chaque amorce C-xN et D-xN, pour chaque amorce A-xN et N-yN, pour chaque amorce M-xN et D-yN et pour chaque amorce M-yN et N-yN par l'équation suivante :

$$\text{Valeur (A-B)}_i = \text{valeur (A-xN)}_i \cdot \text{valeur (B-yN)}_i \ ;$$

$$\text{Valeur (A-D)}_i = \text{valeur (A-xN)}_i \cdot \text{valeur (D-yN)}_i \ ;$$

$$\text{Valeur (C-D)}_i = \text{valeur (C-xN)}_i \cdot \text{valeur (D-yN)}_i \ ;$$

$$\text{Valeur (A-F)}_i = \text{valeur (A-xN)}_i \cdot \text{valeur (F-yN)}_i \ ;$$

$$\text{Valeur (E-D)}_i = \text{valeur (E-xN)}_i \cdot \text{valeur (D-yN)}_i \ ;$$

$$\text{Valeur (E-F)}_i = \text{valeur (E-xN)}_i \cdot \text{valeur (F-yN)}_i \ ;$$

ii) calculer le produit mathématique des valeurs $(A\text{-}B)_i$, $(A\text{-}D)_i$, $(C\text{-}D)_i$ $(A\text{-}F)_i$, $(E\text{-}D)_i$ et $(E\text{-}F)_i$, pour chaque combinaison d'amorces i par l'équation suivante

$$\text{Valeur}^i = \text{valeur (A-B)}_i \cdot \text{valeur (A-D)}_i \cdot \text{valeur (C-D)}_i \cdot \text{valeur (A-F)}_i \cdot \text{valeur (E-D)}_i \cdot \text{valeur (E-F)}_i \ ;$$

iii) comparer les produits mathématiques obtenus $\text{Valeur}^i$, dans lequel les combinaisons d'amorces i avec des valeurs élevées codent pour des conjugués ligand-ADN qui sont présents en concentration élevée dans la bibliothèque codée par ADN.

**15.** Procédé selon la revendication 14, **caractérisé en ce que** le procédé comprend en outre le calcul d'une Valeur[i] par le calcul suivant :

$$\text{Valeur}^i = \log_{10}[\text{valeur}(A\text{-}B)_i \cdot \text{valeur}(A\text{-}D)_i \cdot \text{valeur }(C\text{-}D)_i \cdot \text{valeur}$$
$$(A\text{-}F)_i \cdot \text{valeur }(E\text{-}D)_i \cdot \text{valeur }(E\text{-}F)_i].$$

Figure 1

Figure 2

Figure 3 (top left part thereof)

Q-PCR with A + B

Figure 3 (top right part thereof)

Q-PCR with C+D

Figure 3 (below left part thereof)

## Q-PCR with E + F

Figure 3 (below right part)

## E+F

|  | E3 | E11 | E18 |
|------|-----|------|-----|
| **F3** | 25% | 15% | <1% |
| **F11** | 15% | <1% | 20% |
| **F17** | 10% | 15% | <1% |

Figure 4

EP 3 604 525 B1

Secondary qPCR
A+B

| | | | |
|---|---|---|---|
| B4 | 15% | 10% | 15% | <1% |
| B10 | 10% | <1% | <1% | 10% |
| B17 | <1% | <1% | 15% | 15% |

A2 A6 A11 A17

Secondary qPCR
A+D

| A11B4 | C1D2 |
|---|---|
| A11B17 | C19D2 |

| A17B10 | C1D8 |
|---|---|
| A17B17 | C10D8 |

| A2-B4 | C1D8 |
|---|---|
| A2-B10 | C10D8 |

| A11-B4 | C1D15 |
|---|---|
| A11-B17 | C10D15 |
| | C19D15 |

| A17-B10 | C1D15 |
|---|---|
| A17-B17 | C10D15 |
| | C19D15 |

| | | | |
|---|---|---|---|
| D2 | <1% | <1% | 15% | <1% |
| D8 | 10% | <1% | <1% | 10% |
| D15 | <1% | <1% | 20% | 10% |
| D18 | 15% | <1% | 10% | 10% |

A2 A6 A11 A17

Secondary qPCR
C+D

| | | |
|---|---|---|
| D2 | 5% | <1% | 15% |
| D8 | 10% | 10% | <1% |
| D15 | 10% | 5% | 15% |
| D18 | <1% | 20% | 10% |

C1 C10 C19

| A2-B4 | C10D18 |
|---|---|
| A2-B10 | C19D18 |

| A11B4 | C10D18 |
|---|---|
| A11B17 | C19D18 |

| A17B10 | C10D18 |
|---|---|
| A17B17 | C19D18 |

$$\text{Value}_{\text{building block 1-2}} = \text{Value}_{\text{matrix-A+D}} \cdot \text{Value}_{\text{matrix-A+B}} \cdot \text{Value}_{\text{matrix-C+D}}$$

42

Figure 5

## Figure 6

| | | | | | | |
|---|---|---|---|---|---|---|
| | | | | | | C1D2 |
| | | | | | | C1D8 |
| | | | | | | C10D8 |
| 5% | | | 10% | 10% | | C1D15 |
| | | | | | | C10D15 |
| | | | 15% | 20% | | C19D15 |
| | | 15% | | 5% | | C10D18 |
| | | | | | | C19D18 |
| | | | 10% | 10% | | C19D2 |

A17B17   A17B10   A2B4   A11B4   A11B17   A2B10

A11B17-C19D15
A11B4-C19D15
A2B4-C10D18
A11B4-C19D2
A11B17-C19D2
A11B4-C1D15
A11B17-C1D15

F3E3
F11E18
F3E11
F11E3
F17E11

# Figure 7

Figure 8

A library of $4^{10}$ compounds

Primer

Primer

E ——————— F    Primer for all compounds

E ——————— $F_1$    Primer for 1/4 compounds

E ——————— $F_5$    Primer for 1/4$^5$ compounds

Primer for all compounds

Primer for all compounds

Primer for all compounds

Experimental $\Delta C_q$ between $E_x$ and F

$F_1$    1.8
$F_5$    4.9

Figure 9

Figure 10

| | ΔCq | |
|---|---|---|
| 1a → ← 1b | 13.12 | Full library |
| 2a → ← 1b | 9.98 | a fraction containing CBS |
| 1a → ← 2b | 9.96 | a fraction containing CBS |
| 2a → ← 2b | 7.36 | Compound CBS |
| 3a → ← 1b | 15.04 | a fraction containing Theo |
| 1a → ← 3b | 14.51 | a fraction containing Theo |
| 3a → ← 3b | 14.72 | Compound Theo |

DBC

Figure 11